# EUROPEAN PATENT SPECIFICATION

(11) **EP 4 408 561 B1**
(45) Date of publication and mention of the grant of the patent: **16.04.2025**
(21) Application number: 22789502.6
(22) Date of filing: 14.09.2022
(51) Int. Cl.: B01D 15/18, G01N 30/60

(54) **PURIFICATION METHOD AND USES THEREOF**
REINIGUNGSVERFAHREN UND VERWENDUNGEN DAVON
PROCÉDÉ DE PURIFICATION ET SES UTILISATIONS

(30) Priority: 28.09.2021 EP 21199535
(43) Date of publication of application: 07.08.2024
(73) Proprietor: ChromaCon AG, 8005 Zürich (CH)
(72) Inventor: AUMANN, Lars, 8057 Zürich (CH); WELDON, Richard, 8046 Zürich (CH); VOGG, Sebastian, 4057 Basel (CH); MÜLLER-SPÄTH, Thomas, 8049 Zürich (CH)
(74) Representative: Bremi, Tobias Hans
(86) International application number: PCT/EP2022/075490
(87) International publication number: WO 2023/052126

(56) References cited:
- EP-A1- 2 772 289
- EP-A1- 2 925 419
- WO-A1-2006/116886
- WO-A1-2018/011102
- WO-A1-2019/096622

## Description

### TECHNICAL FIELD

The present invention relates to a cyclic chromatographic method for producing high-purity therapeutics originating from chemical synthesis such as peptides and oligonucleotides.

### PRIOR ART

The purification process of active substances such as therapeutic peptides, oligonucleotides and proteins typically includes a series of chromatographic steps.

In many cases of peptide and oligonucleotide production, the target compound is obtained by chemical synthesis. In this process, apart from the target compound, product-related impurities are generated that need to be removed in the downstream process.

Due to its high selectivity, chromatography is an indispensable unit operation for removal of product-related impurities. The objective of chromatography is producing a product pool that meets purity specifications, while maintaining a high product yield and throughput.

To achieve this aim, linear gradient chromatography is used frequently. In linear gradient chromatography, after binding of the product to the stationary phase in the chromatographic adsorber, the composition if the mobile phase that is pumped through the adsorber is gradually changed over time with a constant slope.

This leads to a sequential desorption of early eluting (weakly adsorbing) impurities, product, and late eluting (strongly adsorbing) impurities. In a chromatogram, this sequence of eluting compounds is visible as a series of peaks. By running a shallower linear gradient, the resolution of the compounds to be separated can be improved, however this comes at an increase in processing time and a reduction in throughput. Likewise, the resolution of the compounds can be increased by reducing the mass of starting material (feed) loaded onto the column, however this reduces the throughput as well.

In preparative chromatography, throughput is of high importance and therefore the gradient duration is limited leading to overlapping impurity and product peaks in preparative chromatograms. The highest product purity is typically obtained in the center of the product peak and this portion of the chromatogram is collected as pool at the adsorber outlet during the elution. Including side fractions where impurity peaks and product peak are overlapping with the product, leads to a reduction in product pool purity. To avoid violation of purity constraints, typically a portion of the product in the side fractions has to be excluded from the product pool. The fraction of product not included in the product pool can even constitute the majority of product contained in the starting material loaded onto the adsorber. To avoid product losses, it is of high interests to recover the product contained in the impure side fractions. Frequently, the side fractions are subject to re-chromatography, i.e. the same or similar chromatographic unit operation is carried out using the side fractions as load material. Through this operation, a fraction of the product can be recovered pure, however the separation is more difficult as the load material has a higher content of impurities than the regular feed material. Re-chromatography also has a series of operational disadvantages including regulatory limitations, side fraction storage and handling, side fraction stability and quality control.

Several processes have been presented to automate the recycling of impure side fractions in chromatography. These processes can be grouped into single- and multi-adsorber recycling processes.

Single adsorber setups include recirculation of the chromatographic profile through the same column. In the steady-state-recycling (SSR) process, fractions are collected from the leading and trailing portions of the circulating chromatographic profile, and fresh sample is injected into the interior of the profile.

Multi-adsorber recycling processes allow combination of internal recycling of impure side-fractions from one adsorber to another and the use of counter-current principles, i.e. a relative opposite movement of stationary phase and mobile phase, which improves the resolution of product and impurities. Through automatic recycling, the generation and collection of side fractions, external storage, handling, and analysis of side-fractions is avoided, while only pure product is recovered from the process with high yield.

Multi-adsorber processes combining internal recycling and counter-current principles are known as simulated moving bed (SMB) processes. Early SMB processes were limited to separations of two compounds (binary separations) and could not operate under linear gradient conditions, limiting their application to separations that did not require a center cut in the chromatogram.

The concept of SMB was further developed to result in a very efficient process for center-cut (ternary) separations with linear solvent gradient capabilities, known as "MCSGP" process (Multicolumn Countercurrent Solvent Gradient Purification), see EP-A-1 877 769 and WO 2019/096622 A1.

This process has been well established in industry. Other chromatographic multi-adsorber techniques using a multitude of adsorbers and internal recycling have been suggested, such as a "gradient with steady state recycle" (GSSR) process.

Although MCSGP is has been described for 2-8 column configurations, in practice mainly the 2-adsorber configuration is used due to lower equipment complexity and higher operational flexibility as opposed to setups using more columns.

The MCSGP process is designed based on a single column batch chromatography chromatogram. Generally, with MCSGP it is possible to obtain product of the purity corresponding to the purest fraction from the batch chromatogram. In some cases, the overlapping impurities may extend far below the product peak, limiting the maximum possible purity to be obtained by MCSGP.

### SUMMARY OF THE INVENTION

The present invention aims at providing a further improved MCSGP like process that can reach higher product purity than a regular MCSGP process.

It was surprisingly found that through introduction of additional recycling phases into process similar to the MCSGP process, combined with a change of the loading scheme, not only a higher purity but also a higher or equal productivity (throughput) for a higher purity can be obtained at comparable yield.

The proposed additional recycling phases transfer the chromatographic profile from one chromatographic adsorber to the other whereby inline dilution is applied so that the chromatographic profile is re-adsorbed on the downstream adsorber. Typically, this in-line dilution is done in a way that eluent with none or a low modifier content is essentially constantly added between the two columns to decrease the elutropic strength of the downstream liquid slowing down elution in the downstream column. Typical modifiers in case of reverse phase chromatography are organic solvents, while in ion exchange typical modifiers are salts (modifying the ionic strength) or acids/bases (modifying the pH). The inline dilution is set to a level where full adsorption of the compounds eluting from the upstream column is expected and it is not intended to promote further separation on the downstream column during the recycling phase. Therefore, it is expected that the partial separation of the compounds in the chromatographic profile, obtained through the elution from the upstream, is annihilated through the inline dilution. In other words, through inline dilution, a loss of separation would be expected leading to the same situation as after feed injection.

Therefore, a person skilled in the art would expect that introduction of a recycling step with normal or "excessive" inline dilution would not lead to an improvement of product purity. Moreover, a person skilled in the art would expect that introduction of recycling steps would lower the overall process productivity (throughput) because recycling steps take extra time and during this time no additional feed is introduced and no product is produced. Surprisingly, it was found that the time loss resulting from the introduction of recycling phases can be overcompensated for by an increase of the load. In addition to that due to the recycling significantly higher purities can be achieved.

The presented method uses two chromatographic adsorber sections as chromatographic stationary phase. One adsorber section may consist of one single column, however it may also consist of several columns, in that case the columns of one section are always interconnected and never disconnected in the process.

The first adsorber section has a first adsorber section inlet and a first adsorber section outlet, and the section second adsorber section has a second adsorber section inlet and a second adsorber section outlet.

The presented method comprises an optional but preferred Startup Phase, a Recycling Phase with one or several recycling sequences (n ≥ 1), a Purification Phase carried out once only and after the Recycling Phase. The Recycling Phase and the Purification Phase form the so called Base Sequence, and this Base Sequence is repeated at least once (m ≥ 1). After the desired number of Base Sequences, there follows an optional but preferred Shutdown Phase.

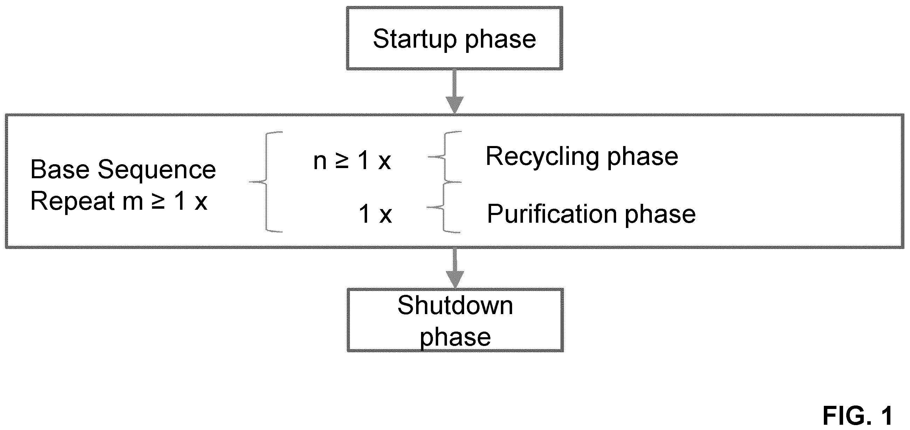

This basic principle is schematically illustrated in **Figure 1****.**

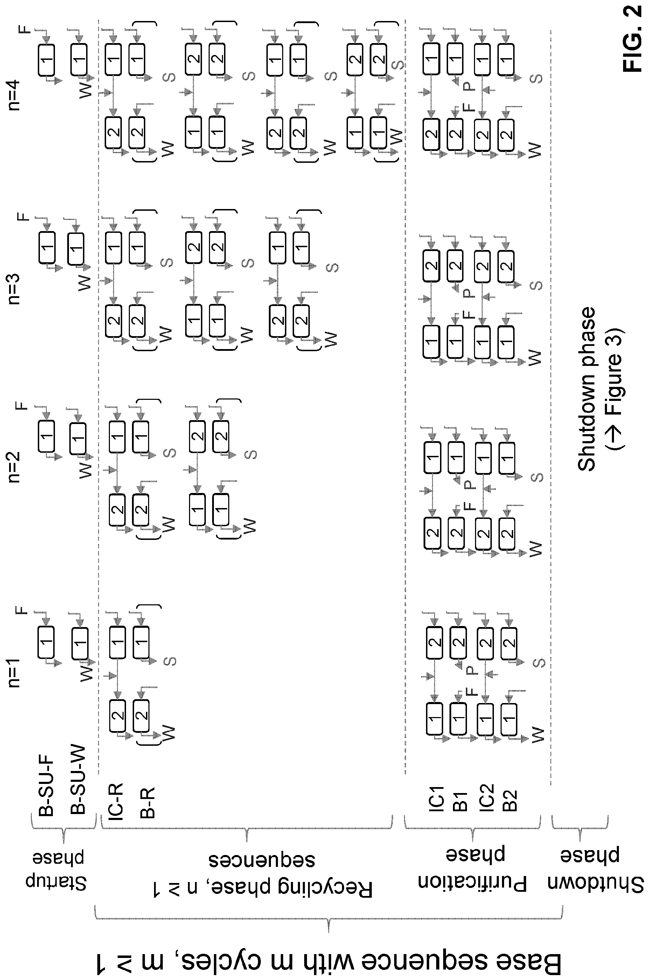

**Figure 2** shows the presented method for different numbers n = 1,2,3,4 of cycles in the Recycling Phase. All examples start with a Startup Phase and end with a Shutdown Phase. In the Startup Phase, the, normally previously equilibrated, first adsorber 1 is loaded with Feed mixture F. The feed mixture comprises the desired product P as well as impurities, namely more weakly adsorbing impurities W and more strongly adsorbing impurities S. Subsequently, the weakly adsorbing impurities W, i.e. impurities which are more weakly adsorbing to the stationary phase than the product P, are eluted from this adsorber with or without (as illustrated and preferred) continuing the Feed flow. Afterwards, the process enters the Recycling Phase.

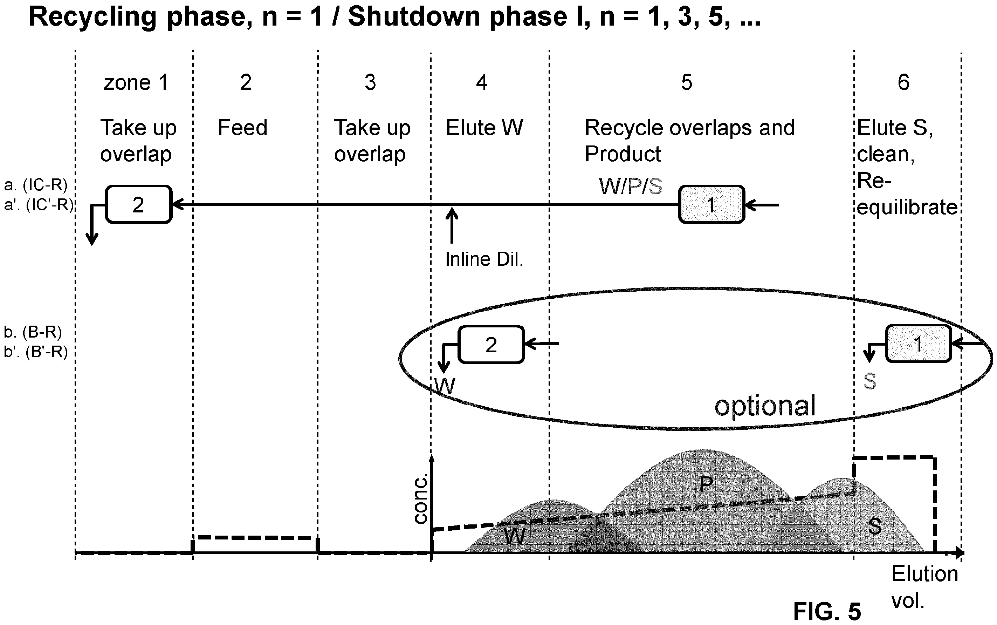

For n=1, the Recycling Phase itself consists of one cycle, i.e. of one interconnected (IC-R) and one disconnected (B-R) step. In the interconnected step, the outlet of the first adsorber 1 is connected to the inlet of the second adsorber 2 while feeding with solvent, preferably with a gradient (see further below, in particular Fig. 5). Inline dilution is performed in between the adsorbers. Inline dilution takes place with the base solvent and thus without gradient.

Then, in the following disconnected step B-R, the adsorbers are disconnected and strongly adsorbing impurities S, i.e. the impurities which are more strongly adsorbing to the stationary phase than the product, are eluted from the first adsorber 1 and this adsorber is re-equilibrated, while weakly adsorbing impurities W are eluted from the second adsorber 2. This latter disconnected step B-R in the recycling phase is optional.

For n=2, the Recycling Phase consists of the Recycling Phase of n = 1, supplemented by one more cycle, i.e. one further interconnected and one further (optional) disconnected step. In the further interconnected step, the outlet of the former second adsorber 2 is connected to the inlet of the former first adsorber 1. Then, in the following further disconnected step, the adsorbers are disconnected and strongly adsorbing S impurities are eluted from the second adsorber 2 and the adsorber is re-equilibrated, while weakly adsorbing W impurities are eluted from the first adsorber 1. This latter further disconnected step is again optional. For n=3, the Recycling Phase consists of the Recycling Phase of n = 2, supplemented by one even further interconnected and one even further (optional) disconnected step. In the interconnected step, the outlet of the first adsorber 1 is connected to the inlet of the second adsorber 2. Then, in the following disconnected step, the adsorbers are disconnected and strongly adsorbing S impurities are eluted from the first adsorber 1 and the adsorber is re-equilibrated, while weakly adsorbing W impurities are eluted from the second adsorber 2. This latter disconnected step is again optional.

For n=4, the Recycling Phase consists of the Recycling Phase of n = 3, supplemented by again one further interconnected and again one further disconnected step. In the interconnected step, the outlet of the second adsorber 2 is connected to the inlet of the first adsorber 1. Then, in the following disconnected step, the adsorbers are disconnected and strongly adsorbing S impurities are eluted from the second adsorber 2 and the adsorber is re-equilibrated, while weakly adsorbing W impurities are eluted from the first adsorber 1. This latter disconnected step is optional.

In general, for any cycle number n (with n > 1), the Recycling Phase consists of the steps and of the Recycling Phase of n-1, supplemented by one further interconnected and one further (optional) disconnected phase.

In general, for even numbers n = 2, 4 6, ..., of the Recycling Phase, in the last interconnected step of the Recycling Phase, the outlet of the second adsorber 2 is connected to the inlet of the first adsorber 1. Then, in the following disconnected step, if carried out, the adsorbers are disconnected and strongly adsorbing S impurities are eluted from the second adsorber 2 and the adsorber is re-equilibrated, while weakly adsorbing W impurities are eluted from the first adsorber 1. This latter disconnected step is optional.

In general, for uneven numbers n =1, 3, 5, ..., of the Recycling Phase, in the last interconnected step of the Recycling Phase, the outlet of the first adsorber 1 is connected to the inlet of the second adsorber 2. Then, in the following disconnected step, the adsorbers are disconnected and strongly adsorbing S impurities are eluted from the first adsorber 1 and the adsorber is re-equilibrated, while weakly adsorbing W impurities are eluted from the second adsorber 2. This latter disconnected step is optional.

The Recycling Phase is followed by a Purification Phase. Each Purification Phase comprises a first interconnected step IC1, a first batch (disconnected) step B1, a second interconnected step IC2 and a second batch (disconnected) step B2.

For n = 1 and n = 3 and any uneven natural numbers n, in the interconnected step IC1 of the Purification Phase, the outlet of the second adsorber 2 is connected to the inlet of the first adsorber 1, i.e., the second adsorber 2 is upstream of the first adsorber 1 and inline dilution is performed between the two adsorbers. In the subsequent batch step B1, the adsorbers are disconnected and purified product P is eluted and collected from the second adsorber 2 while new feed mixture F is loaded onto the first adsorber 1. In the following interconnected step IC2, the adsorbers are connected and the second adsorber 2 is upstream of the first adsorber 1 and inline dilution is performed between the two adsorbers. In the following disconnected step B2, the adsorbers are disconnected and strongly adsorbing S impurities are eluted from the second adsorber 2 and the adsorber is re-equilibrated, while weakly adsorbing W impurities are eluted from the first adsorber 1.

For n = 2 and n = 4 and any even natural numbers n, in the interconnected step IC1 of the Purification Phase, the outlet of the first adsorber 1 is connected to the inlet of the second adsorber 2, i.e., the first adsorber 1 is upstream of the second adsorber 2 and inline dilution is performed between the two adsorbers. In the subsequent Batch step B1, the adsorbers are disconnected and purified product P is eluted and collected from the first adsorber 1 while new feed mixture F is loaded onto the second adsorber 2. In the following Interconnected step IC2, the adsorbers are connected and the first adsorber 1 is upstream of the second adsorber 2 and inline dilution is performed between the two adsorbers.

In the following disconnected step, the adsorbers are disconnected and strongly adsorbing S impurities are eluted from the first adsorber 1 and the adsorber is re-equilibrated, while weakly adsorbing W impurities are eluted from the second adsorber 2.

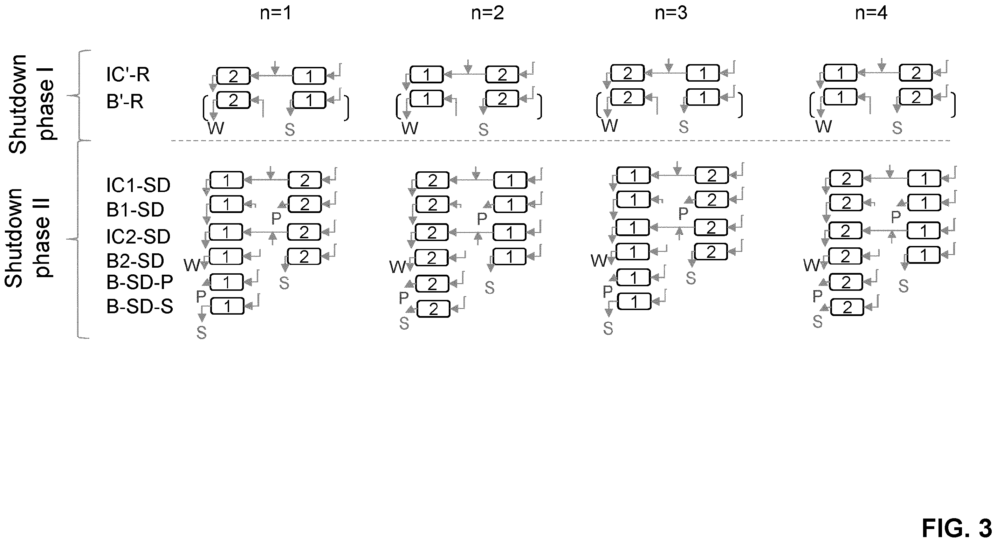

For any numbers n of Recycling Phases and after the desired number m of base sequences, the last Purification Phase is followed by a Shutdown Phase that is described in Fig 3.

**Figure 3** shows the Shutdown Phases for the different numbers n = 1,2,3,4,... of Recycling Phases. The last Purification Phase of the desired number of base sequences is followed by a Shutdown phase that itself consists of two phases, Shutdown Phases I and II.

The first phase, Shutdown Phase I, consists of the same steps as the last IC-R and (optional) B-R steps of the preceding Recycling Phase of the process.

The second shutdown phase, Shutdown Phase II, consists of the same steps as the previous Purification Phase of the process with some modification, but is extended by additional steps to elute product P and to elute impurities S, but it is operated without further loading of new feed mixture F.

For n=1 and n = 3 and any uneven natural numbers n, the Shutdown Phase I consists of an interconnected step, wherein the outlet of the first adsorber 1 is connected to the inlet of the second adsorber 2. Inline dilution is performed in between the adsorbers. In the following disconnected step, the adsorbers are disconnected and strongly adsorbing S impurities are eluted from the first adsorber 1 and the adsorber is re-equilibrated, while weakly adsorbing W impurities are eluted from the second adsorber 2. This latter disconnected step is optional.

For n=1 and n = 3 and any uneven natural numbers n, the Shutdown Phase II consists of an interconnected step IC1, in which the outlet of the second adsorber 2 is connected to the inlet of the first adsorber 1, i.e., the second adsorber 2 is upstream of the first adsorber 1 and inline dilution is performed between the two adsorbers. In the subsequent Batch step B1, the adsorbers are disconnected and purified product P is eluted and collected from the second adsorber 2. In this step, no new feed mixture F is loaded onto the first adsorber 1. In the following Interconnected step IC2, the adsorbers are connected and the second adsorber 2 is upstream of the first adsorber 1 and inline dilution is performed between the two adsorbers. Subsequently purified product P is eluted and collected from the first adsorber 1 and strongly adsorbing impurities S are eluted form the first adsorber 1.

For n=2 and n = 4 and any even natural numbers n, the Shutdown Phase I consists of the same steps as the Shutdown Phase I for uneven numbers of n, but adsorbers 1 and 2 are interchanged, and for n=2 and n = 4 and any even natural numbers n, the Shutdown Phase II consists of the same steps as the Shutdown Phase II for uneven numbers of n, but adsorbers 1 and 2 are interchanged.

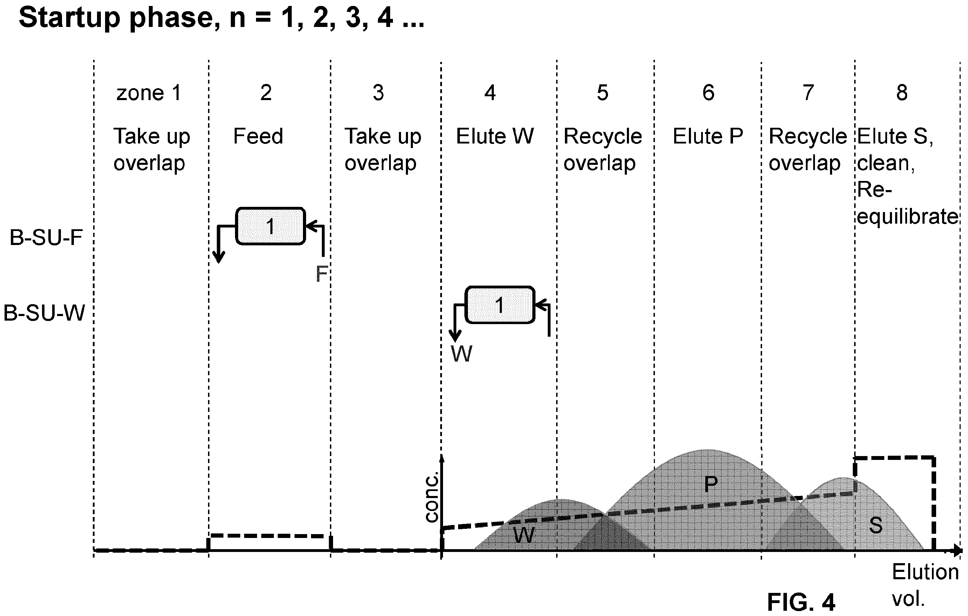

**Figure 4** shows the tasks of the two adsorbers in the Startup Phase for any number n. In the Startup Phase, the previously equilibrated first adsorber 1 is loaded with Feed mixture F (step B-SU-F). Subsequently, weakly adsorbing impurities W are eluted from this adsorber (step B-SU-W). The linear gradient segment, flow rate and switch time to be operated in the first adsorber 1 in step B-SU-W preferably correspond to what is chosen for a single column chromatogram.

Generally, the operating parameters, i.e. the flow rates, gradient concentrations, feed amount and switch time of the presented method can be derived from a single adsorber chromatogram, as shown at the bottom of Fig. 4. Thus, the presented method can be designed and initially set up based on a single adsorber chromatogram, further more refined control during operation based on e.g. detector feedback being possible.

**Figure 5** shows the Recycling Phase of the presented method for n = 1.

The Recycling Phase itself comprises at least of one sequence of interconnected (IC-R) and disconnected (B-R) steps. In the first interconnected step (IC-R), the outlet of the first adsorber 1 is connected to the inlet of the second adsorber 2. Adsorbed compounds W, P, S are eluted from the first adsorber 1 into the second adsorber 2 using a solvent gradient, wherein inline dilution is performed in between the adsorbers with base solvent without modifier or with low modifier content such that W, P, S are strongly adsorbed on the second adsorber 2 and preferably not even weakly adsorbing W impurities exit the downstream adsorber 2. Then, in the following first disconnected step (B-R), the adsorbers are disconnected and remaining S impurities are eluted from the first adsorber 1 using a high, preferably constant modifier concentration, and then the adsorber 1 is re-equilibrated, while weakly adsorbing W impurities are eluted from the second adsorber 2 using a gradient with low starting modifier concentration (see dashed line on the bottom below the respective step indicating the modifier concentration as a function of the process). This latter disconnected step B-R is optional, and its importance depends on the purity of the product P in the feed mixture F and the purity specification. A lower purity corresponds to a larger content of W and S and therefore one would consider including the disconnected step (B-R) in the Recycling Phase for removal of W and S. Again, the operating parameters, i.e. the flow rates, gradient concentrations, feed amount and switch time of the presented method can be derived from a single adsorber chromatogram, as shown at the bottom of Fig 5, further more refined control during operation based on e.g. detector feedback being possible.

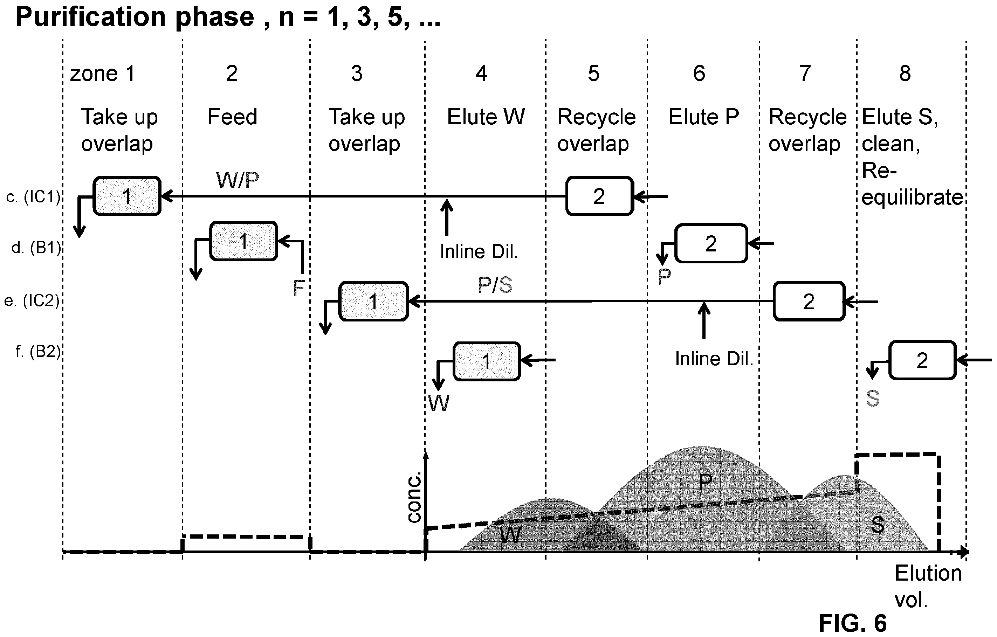

For n=1, the Shutdown Phase I is identical to the Recycling Phase. Each Recycling Phase is followed by a Purification Phase (Fig. 2, Fig. 6)
**Figure 6** shows the Purification Phase of the presented method for n = 1, 3, 5 ...

Each Purification Phase comprises a first interconnected step (IC1), a first batch (disconnected) step (B1), a second interconnected step (IC2) and a second batch (disconnected) step (B2).

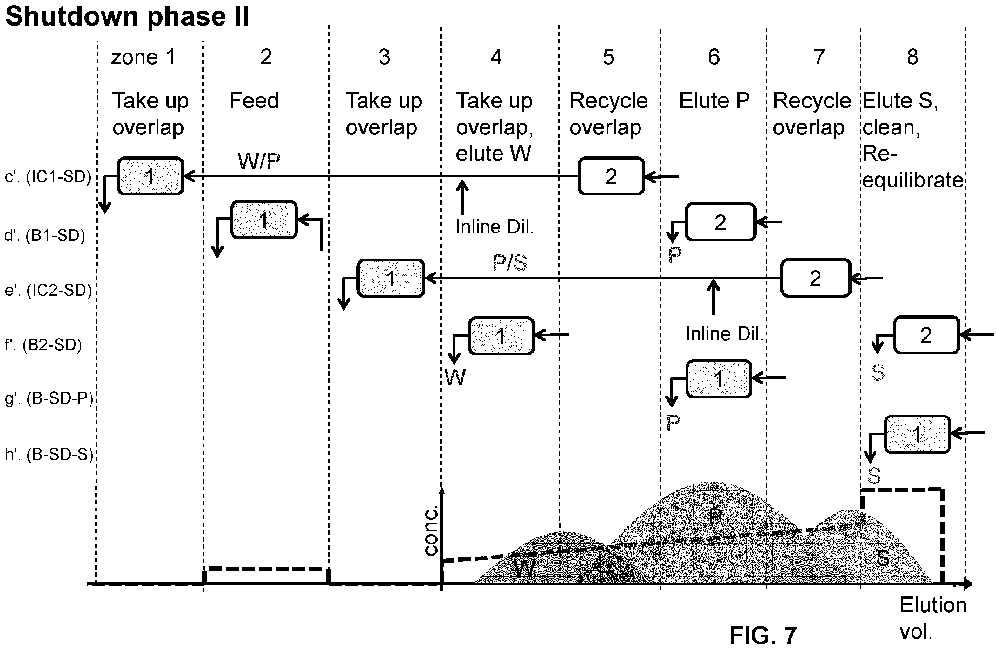

In the interconnected step IC1, the outlet of the second adsorber 2 is connected to the inlet of the first adsorber 1, i.e. the second adsorber 2 is upstream of the first adsorber 1. In the interconnected step IC1, a partially pure side fraction containing W and P (overlapping region in the chromatogram, see schematic chromatogram at bottom of Fig. 6 ("zone 5") is eluted from the second adsorber 2 into the first adsorber 1. During IC1 operation, the stream containing W and P is diluted inline before entering the first adsorber 1 such that W and P are fully adsorbed at the entrance of the first adsorber 1. In the subsequent Batch step B1, the adsorbers are disconnected and purified product P is eluted and collected from the second adsorber 2, while new feed mixture F is loaded onto the first adsorber 1. In the following Interconnected step IC2, the adsorbers are connected and the second adsorber 2 is upstream of the first adsorber 1 and a partially pure side fraction containing P and S (overlapping region in the chromatogram, see bottom of Fig. 6 ("zone 7")) is eluted from the second adsorber 2 into the first adsorber 1. The stream containing P and S is inline diluted before entering the first adsorber 1 such that the compounds P and S are fully adsorbed at the entrance of the first adsorber 1. Again, the operating parameters, i.e. the flow rates, gradient concentrations, feed amount and switch time of the presented method can be derived from a single adsorber chromatogram, as shown at the bottom of Fig. 6, further more refined control during operation based on e.g. detector feedback being possible. **Figure 7****:** To avoid product P losses at the end of the process operation, the Purification phase is followed by a Shutdown Phase that itself consists of two phases, a Shutdown Phase I, and a Shutdown Phase II.

For n=1, 3, 5 ..., the Shutdown Phase I is functionally identical to the Recycling Phase for n=1 and shown in Fig. 5. Shutdown Phase I comprises the steps IC'-R and B'-R using the same conditions as the last Sequence of the Recycling Phase of the process.

Fig. 7 shows the Shutdown Phase II of the presented method for n = 1, 3, 5, ....

The Shutdown Phase II comprises essentially the same steps as the previous Purification Phase of the method but without feed, see Fig. 6 (IC1-SD corresponding to IC1, B1-SD corresponding to B1 but without feed, IC2-SD corresponding to IC2, B2-SD corresponding to B2) but is extended by additional steps to elute product P (B-SD-P) from the first adsorber 1 that previously was in the downstream position and to elute impurities S and to clean and regenerate the same adsorber (B-SD-S). The conditions of in B-SD-P and B1-SD for collection of P are preferably the same in terms of the flow rates and gradient concentrations. Likewise, the conditions of in B-SD-S and B2-SD for removal of S are preferably the same in terms of the flow rates and gradient concentrations.

In case the preceding Recycling Phase comprised an even number of sequences (n=2,4,6 ...) of interconnected steps IC-R and disconnected steps B-R, the positions of the adsorbers in the steps IC1-SD, B1-SD, IC2-SD, and B2-SD is exchanged, i.e. in the interconnected step IC1-SD, the outlet of the first adsorber 1 is connected to the inlet of the second adsorber 2, such that the first adsorber 1 is upstream of the second adsorber 2. Likewise, the positions of the adsorbers in the phases B1-SD, IC2-SD, and B2-SD are exchanged. In the presented process, gradients, in particular linear gradients may be and preferably will be used in any steps to enhance separation of the compounds and/or modify the speed of elution by changing the gradient slope.

Selecting a higher gradient slope in the Recycling Phase steps (IC-R and/or B-R) than for the Purification Phase steps (IC1, B1, IC2, and/or B2) allows the Recycling Phase to be completed faster and therefore will improve overall process productivity.

More generally speaking, the present invention relates to a cyclic chromatographic purification method for the isolation of a product P from a feed mixture F consisting of the product P and at least two further components representing weakly adsorbing impurities W and strongly adsorbing impurities S.

The proposed method uses only two chromatographic adsorber sections as chromatographic stationary phase. A first adsorber section has a first adsorber section inlet and a first adsorber section outlet, and a second adsorber section has a second adsorber section inlet and a second adsorber section outlet.

The proposed method comprises at least one Base sequence having at least one Recycling Phase followed by only one Purification Phase, wherein preferably Base sequences are repeated in a cyclic manner at least twice.

According to the invention, said Recycling Phase consists of at least one recycling sequence, preferably of a sequence of only one or at least two, or at least three, or at least four, recycling sequences, comprising the following steps:
a. an interconnected recycling step IC-R, in which the adsorber sections are interconnected, and in which an upstream adsorber section outlet is connected to a downstream adsorber section inlet during a recycling interconnected timespan t_{IC-R}, wherein the upstream adsorber section is loaded via the upstream adsorber section inlet with eluent and wherein a fraction containing the weakly adsorbing impurities W at least as far as overlapping with product P, the product P, and the strongly adsorbing impurities S at least as far as overlapping with product P is eluted from the upstream adsorber section into the downstream adsorber section and
   wherein the stream exiting the upstream adsorber outlet is diluted inline before entering the downstream adsorber inlet;
b. an optional batch recycling step B-R, wherein the during a recycling batch timespan t_{B-R} said adsorber sections are disconnected and
   wherein the previously upstream adsorber section of preceding step a. is cleaned to remove the strongly adsorbing impurities S and regenerated and wherein eluent is loaded to the inlet of the previously downstream adsorber section of step a. to elute weakly adsorbing impurities W as far as not overlapping with product P;
wherein after each recycling sequence of steps a. and b., the adsorber sections are switched in order, wherein the number of recycling sequences is ≥ 1.

According to the invention, the Recycling Phase is followed by only one Purification Phase. This Purification Phase comprises the following steps in order, wherein the upstream adsorber section of the last interconnected recycling step of the preceding Recycling Phase takes the function of the downstream adsorber section and the downstream adsorber section of the last interconnected recycling step of the preceding Recycling Phase takes the function of the upstream adsorber section:
c. a first interconnected purification step IC1, in which the adsorber sections are interconnected, wherein the upstream adsorber section outlet is connected to the downstream adsorber section inlet during a first interconnected purification timespan t_{IC1},
   wherein the upstream adsorber is loaded via the upstream adsorber inlet with eluent and wherein a product fraction containing weakly adsorbing impurities W and Product P in overlap is eluted from the upstream adsorber into the downstream adsorber section and
   wherein the stream exiting the upstream adsorber section outlet is diluted inline before entering the downstream adsorber section inlet
d. a first batch purification step B1, wherein during a first batch purification timespan t_{B1} said adsorber sections are disconnected and wherein product P is eluted from the previous upstream adsorber section and wherein feed mixture F is supplied to the previous downstream adsorber section inlet
e. a second interconnected purification step IC2, wherein the upstream adsorber section outlet is connected to the downstream adsorber section inlet during a second interconnected purification timespan t_{IC2},
   wherein the upstream adsorber section is loaded via the upstream adsorber section inlet with eluent and wherein a product fraction containing Product P and strongly adsorbing impurities S in overlap is eluted from the upstream adsorber into the downstream adsorber and
   wherein the stream exiting the upstream adsorber section outlet is diluted inline before entering the downstream adsorber section inlet
f. a second batch purification step B2, wherein during a second purification batch timespan tB2 said adsorbers are disconnected and wherein the previous upstream adsorber is cleaned and regenerated and wherein eluent is loaded to the previous downstream adsorber inlet.

As pointed out above, preferably eluent gradients are used in at least one or all of the phases.

In interconnected steps of the method preferably the upstream section inlet is loaded via the upstream adsorber inlet with eluent with a gradient in the form of a temporally changing modifier concentration and the stream exiting the upstream adsorber section outlet is diluted inline before entering the downstream adsorber section inlet with eluent without modifier or with a different, preferably lower, modifier concentration than at the inlet of the upstream adsorber section.

Further preferably, in batch steps of the method without purified product elution the previous upstream adsorber is cleaned, e.g. with eluent with a higher modifier concentration than at the end of the preceding interconnected recycling step or with a different modifier or with a cleaning solution, and regenerated and wherein eluent with a gradient in the form of a temporally changing modifier concentration is loaded to the previous downstream adsorber inlet.

In batch steps of the method with purified product elution preferably the previous upstream adsorber is loaded via the upstream adsorber inlet with eluent with a gradient in the form of a temporally changing modifier concentration.

In said Recycling Phase in said interconnected recycling step IC-R, according to a preferred embodiment the upstream adsorber section is loaded via the upstream adsorber section inlet with eluent with a gradient in the form of a temporally changing modifier concentration, and the stream exiting the upstream adsorber outlet is diluted inline before entering the downstream adsorber inlet with eluent without modifier or with a different modifier concentration than at the inlet of the upstream adsorber section.

According to yet another preferred embodiment, in the optional batch recycling step B-R, the previously upstream adsorber section of preceding step a. is cleaned with eluent, e.g. with a higher modifier concentration than at the end of the preceding interconnected recycling step or with a different modifier, or with a cleaning solution, and regenerated and eluent with a gradient in the form of a temporally changing modifier concentration is loaded to the inlet of the previously downstream adsorber section of step a. to elute weakly adsorbing impurities W as far as not overlapping with product P.

Yet another preferred embodiment is characterised in that in said Purification Phase in said first interconnected purification step IC1 the upstream adsorber section is loaded via the upstream adsorber inlet with eluent with a gradient in the form of a temporally changing modifier concentration and the stream exiting the upstream adsorber section outlet is diluted inline before entering the downstream adsorber section inlet with eluent without modifier or with a different modifier concentration than at the inlet of the upstream adsorber section. Furthermore it is preferred if in said first batch purification step B1 product P is eluted from the previous upstream adsorber section by loading it via its inlet with eluent with a gradient in the form of a temporally changing modifier concentration.

In said second interconnected purification step IC2 preferably said upstream adsorber section inlet is loaded via the upstream adsorber inlet with eluent with a gradient in the form of a temporally changing modifier concentration and the stream exiting the upstream adsorber section outlet is diluted inline before entering the downstream adsorber section inlet with eluent without modifier (base solvent alone) or with a different modifier concentration than at the inlet of the upstream adsorber section.

Also it is preferred if in said second batch purification step B2 the previous upstream adsorber is cleaned with eluent with a higher modifier concentration than at the end of the preceding interconnected recycling step or with a different modifier, or with a cleaning solution, and regenerated and wherein eluent with a gradient in the form of a temporally changing modifier concentration is loaded to the previous downstream adsorber inlet. The modifier is preferably selected from the group consisting of an organic or inorganic solvent (or a mixture thereof) different from a base solvent (or mixture thereof) of the eluent, an electrolyte in such an organic or inorganic solvent (or a mixture thereof), preferably selected from a dissolved salt or a pH, or a combination thereof.

Preferably said base solvent is an organic or inorganic solvent (or a mixture thereof), in particular water or a mixture of water with at least one organic solvent, optionally containing buffer salts, acids, or bases or combinations thereof. For example the base solvent can be a mixture of water 99.9% and trifluoroacetic acid (TFA, 0.1%), and the modifier can be a mixture of 9.9% water, TFA 0.1%, and 90.0% Acetonitrile. Alternatively the modifier can be 100% acetonitrile. In another example the base solvent can be an aqueous 25 mM phosphate buffer, pH 7.0, and the modifier can be a 25 mM phosphate buffer, 500 mM NaCl, pH 7.0.

The base solvent is, in particular in case of biomolecules to be separated, for example resulting from a biochemical process, normally water or water in a mixture with one or more salts and/or organic solvents in a minor proportion compared with water (for example supplemented with acetonitrile and trifluoroacetic acid), in the following this base solvent will be called solvent A. For establishing the gradient this is mixed with a further solvent or solvent mixture different from the base solvent (mixture). This further solvent for example can be a mixture of the same solvents as the base solvent but having different proportions (e.g. for the above example water supplemented with a higher proportion of acetonitrile). In particular for biomolecules typically this further solvent is again based on water but has a further increased proportion of organic solvents. For establishing the gradient typically a modifier mixture with a rather low concentration of the constituent (e.g. organic solvent(s), a salt or pH, or a combination thereof, if e.g. the base solvent is water) differing from the base solvent is provided as eluent at the beginning of the gradient and is increasingly mixed by means of a gradient pump with the further solvent leading to a corresponding controlled gradient. The feed mixture can be provided in a different solvent, or it can be provided in the base solvent or the feed mixture can be provided as a mixture of the original solution (typically a water solution) in a mixture with the base solvent of appropriate concentration. Preferably linear eluent gradients are used in at least one or all of the phases with a gradient in the form of a temporally changing modifier concentration increase.

As mentioned above, preferably before carrying out the first interconnected recycling step of the first Recycling Phase, a Start-up Phase is carried out, in which during a first batch start-up timespan t_{B-SU-F} said adsorbers are disconnected
and feed mixture F is supplied to inlet of the adsorber section to become the upstream adsorber section of the first interconnected recycling step of the first Recycling Phase, while the adsorber section to become the downstream adsorber section of the first interconnected recycling step of the first Recycling Phase is either being equilibrated or already equilibrated and inactive,
and wherein preferably subsequently, during a second batch start-up timespan t_{B-SU-W} said adsorbers are disconnected and weakly adsorbing impurity W is eluted from the adsorber section having been supplied with feed mixture F in the preceding first batch start-up step while the other adsorber is either being equilibrated or already equilibrated and inactive.

After said first batch start-up timespan t_{B-SU-F} and before said second batch start-up timespan (t_{B-SU-W}) eluent can be supplied to the inlet of the adsorber section to become the upstream adsorber section of the first interconnected recycling step of the first Recycling Phase, and said eluent can be without modifier (base solvent alone) or with a modifier concentration essentially corresponding to the starting modifier concentration applied during said first batch start-up timespan or in the absence of a second batch start-up step of the first interconnected recycling step.

Further during said second batch start-up timespan the inlet of the adsorber section having been supplied with feed mixture F can be loaded with eluent with a gradient in the form of a temporally changing modifier concentration.

After termination of at least one base sequence, preferably more than one base sequences, a Shut-down Phase can be carried out, wherein the Shutdown Phase comprises the following steps in order:
a'. an interconnected shutdown recycling step IC'-R, in which the upstream adsorber section of the preceding and last second interconnected purification step IC2 takes the downstream position and the other adsorber section takes the upstream position, in which the adsorber sections are interconnected, and in which the upstream adsorber section outlet is connected to the downstream adsorber section inlet during a shutdown recycling interconnected timespan t_{IC'-R-SD},
   wherein the upstream adsorber section is loaded via the upstream adsorber section inlet with eluent and wherein a fraction containing the weakly adsorbing impurities W at least as far as overlapping with product P, the product P, and the strongly adsorbing impurities S at least as far as overlapping with product P is eluted from the upstream adsorber section into the downstream adsorber section and
   wherein the stream exiting the upstream adsorber outlet is diluted inline before entering the downstream adsorber inlet;
b'. an optional batch shutdown recycling step B'-R, in which the adsorber sections are disconnected, wherein during a shutdown recycling batch timespan t_{B'-R} said adsorber sections are disconnected and
   wherein the previously upstream adsorber section of preceding step a'. is cleaned and regenerated and wherein eluent is loaded to the inlet of the previously downstream adsorber section of step a to elute weakly adsorbing impurities W in as far as not overlapping with product P.

Preferably this is followed by the following steps in order, wherein the upstream adsorber section of the interconnected shutdown recycling step takes the function of the downstream adsorber section and the downstream adsorber section of the interconnected shutdown recycling step takes the function of the upstream adsorber section:
c'. a first interconnected shutdown purification step IC1-SD, in which the adsorber sections are interconnected, wherein the upstream adsorber section outlet is connected to the downstream adsorber section inlet during a first interconnected shutdown purification timespan t_{IC1'},
   wherein the upstream adsorber is loaded via the upstream adsorber inlet with eluent and wherein a product fraction containing weakly adsorbing impurities W and Product P in overlap is eluted from the upstream adsorber into the downstream adsorber section and
   wherein the stream exiting the upstream adsorber section outlet is diluted inline before entering the downstream adsorber section inlet
d'. a first batch shutdown purification step B1-SD, wherein during a first batch purification timespan t_{B1'} said adsorber sections are disconnected and wherein product P is eluted from the previous upstream adsorber section and wherein the previous downstream adsorber section is either idle or eluent is supplied to the previous downstream adsorber section inlet
e'. a second interconnected shutdown purification step IC2-SD, wherein the upstream adsorber section outlet is connected to the downstream adsorber section inlet during a second interconnected shutdown purification timespan t_{IC2'},
   wherein the upstream adsorber section is loaded via the upstream adsorber section inlet with eluent and wherein a product fraction containing Product P and strongly adsorbing impurities S in overlap is eluted from the upstream adsorber into the downstream adsorber and
   wherein the stream exiting the upstream adsorber section outlet is diluted inline before entering the downstream adsorber section inlet
f'. a second batch shutdown purification step B2-SD, wherein during a second purification batch timespan t_{B2'} said adsorbers are disconnected and wherein the previous upstream adsorber is cleaned and regenerated and wherein eluent is loaded to the previous downstream adsorber inlet.

This is typically followed by
g'. a first final shutdown batch step B-SD-P, wherein during a first final shutdown batch timespan t_{B-SD-P} said first and second adsorbers are disconnected and wherein product P is eluted from the previously downstream adsorber and wherein the other adsorber is idle or regenerated
h'. optionally a second final shutdown batch step (B-SD-S), wherein during a second final shutdown batch timespan t_{B-SD-S} said first and second adsorbers are disconnected and strongly adsorbing impurity S is eluted from the previously downstream adsorber and wherein the other adsorber is idle or regenerated.

In said interconnected shutdown recycling step IC'-R, the upstream adsorber section of the preceding and last second interconnected purification step IC2 can be loaded via the upstream adsorber section inlet with eluent of constant composition or with a gradient in the form of a temporally changing modifier concentration, wherein the stream exiting the upstream adsorber outlet is diluted inline before entering the downstream adsorber inlet with eluent without modifier or with a different modifier concentration than at the inlet of the upstream adsorber section.

In said batch shutdown recycling step B'-R the previously upstream adsorber section of preceding step a. can be cleaned with eluent with a higher modifier concentration than at the end of the preceding interconnected recycling step, or with a different modifier or with a cleaning solution, and regenerated and wherein eluent with a gradient in the form of a temporally changing modifier concentration is loaded to the inlet of the previously downstream adsorber section of step a'. to elute weakly adsorbing impurities W in as far as not overlapping with product P.

In said first interconnected shutdown purification step IC1-SD the upstream adsorber can be loaded via the upstream adsorber inlet with eluent with a gradient in the form of a temporally changing modifier concentration and the stream exiting the upstream adsorber section outlet is diluted inline before entering the downstream adsorber section inlet with eluent without modifier or with a different modifier concentration than at the inlet of the upstream adsorber section.

In said first batch shutdown purification step B1-SD product P can be eluted from the previous upstream adsorber section by loading via the upstream adsorber inlet with eluent with a gradient in the form of a temporally changing modifier concentration and the previous downstream adsorber section is either idle or eluent is supplied to the previous downstream adsorber section inlet, preferably the base solvent..

In said second interconnected shutdown purification step IC2-SD the upstream adsorber section can be loaded via the upstream adsorber section inlet with eluent of constant composition or with a gradient in the form of a temporally changing modifier concentration and the stream exiting the upstream adsorber section outlet is diluted inline before entering the downstream adsorber section inlet with eluent without modifier or with a different modifier concentration than at the inlet of the upstream adsorber section.

Further in said second batch shutdown purification step B2-SD, the previous upstream adsorber can be cleaned with eluent with a higher modifier concentration than at the end of the preceding interconnected recycling step and regenerated and wherein eluent is loaded to the previous downstream adsorber inlet.

Finally, in said first final shutdown batch step B-SD-P product P can be eluted from the previously downstream adsorber with eluent with a gradient in the form of a temporally changing modifier concentration and wherein the other adsorber is idle or regenerated and/or in said first final second final shutdown batch step B-SD-S, strongly adsorbing impurity S can be eluted from the previously downstream adsorber with eluent with a higher modifier concentration than at the end of the preceding interconnected recycling step and wherein the other adsorber is idle or regenerated.

Linear gradients with different slopes and/or flow rates are preferably used in the Recycling Phase and/or in the Purification Phase, wherein preferably higher slopes and/or higher flow rates are used in the recycling phase than in the purification phase.

According to yet another preferred embodiment, in most (in particular in all but the steps IC2 and IC2-SD) or all interconnected steps of the method the upstream section inlet is loaded via the upstream adsorber inlet with eluent with a gradient in the form of a temporally changing modifier concentration and the stream exiting the upstream adsorber section outlet is diluted inline before entering the downstream adsorber section inlet with eluent without modifier or with a different modifier concentration than at the inlet of the upstream adsorber section,
and
in batch steps of the method without purified product elution the previous upstream adsorber is cleaned with eluent with a higher modifier concentration than at the end of the preceding interconnected recycling step and regenerated and, if applicable, eluent with a gradient in the form of a temporally changing modifier concentration is loaded to the previous downstream adsorber inlet
or in batch steps of the method with purified product elution the previous upstream adsorber is loaded via the upstream adsorber inlet with eluent with a gradient in the form of a temporally changing modifier concentration

and wherein the modifier is selected from the group consisting of a solvent or mixture thereof different from a base solvent or mixture thereof of the eluent, an electrolyte in such a solvent or mixture thereof, preferably selected from a dissolved salt or a pH, or a combination thereof, wherein preferably said base solvent is water or a mixture of water with at least one organic solvent or water in a mixture with one or more salts and/or organic solvents in a minor proportion compared with water, and wherein further preferably said modifier is an organic solvent or a mixture of water with at least one organic solvent having a higher concentration of said at least one organic solvent than in the base solvent, water or a mixture of water with different salt and/or H⁺ concentration than in the base solvent,
and wherein said modifier concentration is increased, preferably linearly increased, in the process from a lowest concentration at the beginning of the elution (B-SU-W, B-R, B'-R, B2, B2-SD) of the weakly adsorbing impurities W as far as not overlapping with product P, over the following steps of
recycling (IC-R, IC'-R, IC1, IC1-SD), except for the case of a following final shutdown batch step (B-SD-P) and
if applicable, of product elution (B1, B1-SD, B-SD-P) and, if applicable, further recycling (IC2, IC2-SD), wherein during said recycling (IC2, IC2-SD) the modifier concentration can be varied or just for the recycling step be kept constant
to a highest concentration before the beginning of the elution (B-R, B'-R, B2, B2-SD, B-SD-S) of the strongly adsorbing impurities S.

In the presented process, it is possible to use adsorbers of different dimensions or with different stationary phases or both. Elution of Product P is always taking place from the same adsorber, likewise load of new feed material. For example, in case of the Recycling phase comprising only a single sequence of IC-R and B-R (n=1), the main Purification tasks are carried out by adsorber 2 (recycling of W/P, product P elution, recycling of P/S). Therefore, a small particle chromatography resin could be used for this task in adsorber 2 to maximize resolution, while a large particle resin could be used to receive the recycled streams in adsorber 1. Due to inline dilution of W/P and P/S in the steps IC1 and IC2, the flow of eluent entering adsorber 1 is larger than the stream exiting adsorber 2 and therefore adsorber 1 could benefit from larger particles which have a lower backpressure under flow than small particle resins.

Furthermore, the present invention relates to the use of a as detailed above for the purification of biomolecules, of natural or synthetic origin, preferably selected from the group consisting of nucleic acid molecules, including DNA and RNA molecules, proteins, including antibodies, peptides, carbohydrates, lipids as well as combinations and modifications as well as fragments thereof.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a general scheme of the presented process including Startup Phase, Recycling Phase, Purification Phase, and Shutdown Phase;
- Fig. 2: shows the logic of the presented process (Startup Phase, Recycling Phase, Purification Phase) in greater detail including adsorber connections and streams entering and exiting the adsorbers for n=1...4 Sequences in the Recycling Phase. Streams of feeds/eluates not containing W, P, S or F are not marked specifically;
- Fig. 3: shows the logic of the presented process (Shutdown Phase) in greater detail including adsorber connections and streams entering and exiting the adsorbers for n=1...4 Sequences in the Recycling Phase. Streams of feeds/eluates not containing W, P, S or F are not marked specifically;
- Fig. 4: shows the tasks of the two adsorbers in the Startup Phase with reference to a single column chromatogram (shown at the bottom) and schematically indicates linear gradient segments (thick dashed lines) to be used in the process in case of linear gradient operation. The vertical thin dashed lines schematically indicate the section borders; in Figs. 4-7 vertical sections designated as zones and separated by thin vertical dashed lines indicate the different functions of the columns being in the respective zone; on the bottom on the right hand side the chromatogram is illustrated with the product P, and with the more weakly adsorbing impurities W on the left side of the product P eluting earlier than the product P, and with the more strongly adsorbing impurities S on the right side of the product P eluting later than the product P; also given on the bottom in the zone 2 as a dashed line the feed concentration is indicated which is applicable in that zone 2 at the inlet of the column in that zone. In the zones of the chromatogram (zones 4-8 in Fig. 4 and Figs. 6-7 and zones 4-6 in Fig. 5) the dashed line on the bottom indicates the concentration of the modifier which is used for the gradient applied at the inlet of the columns being in the respective zone during elution of the more weakly adsorbing impurities W and of the product P (zones 4-7 in Fig. 4 and Figs. 6-7 and zones 4-5 in Fig. 5), and the high level but normally constant modifier concentration applied in the phase of elution of S without overlap with P (zone 8 in Fig. 4 and Figs. 6-7 and zone 6 in Fig. 5);
- Fig. 5: shows the tasks of the two adsorbers in the Recycling Phase with reference to a single column chromatogram (shown at the bottom) and schematically indicates linear gradient segments to be used in the process in case of linear gradient operation;
- Fig. 6: shows the tasks of the two adsorbers in the Purification Phase with reference to a single column chromatogram (shown at the bottom) and schematically indicates linear gradient segments to be used in the process in case of linear gradient operation;
- Fig. 7: shows the tasks of the two adsorbers in the Shutdown Phase II with reference to a single column chromatogram (shown at the bottom) and schematically indicates linear gradient segments to be used in the process in case of linear gradient operation;

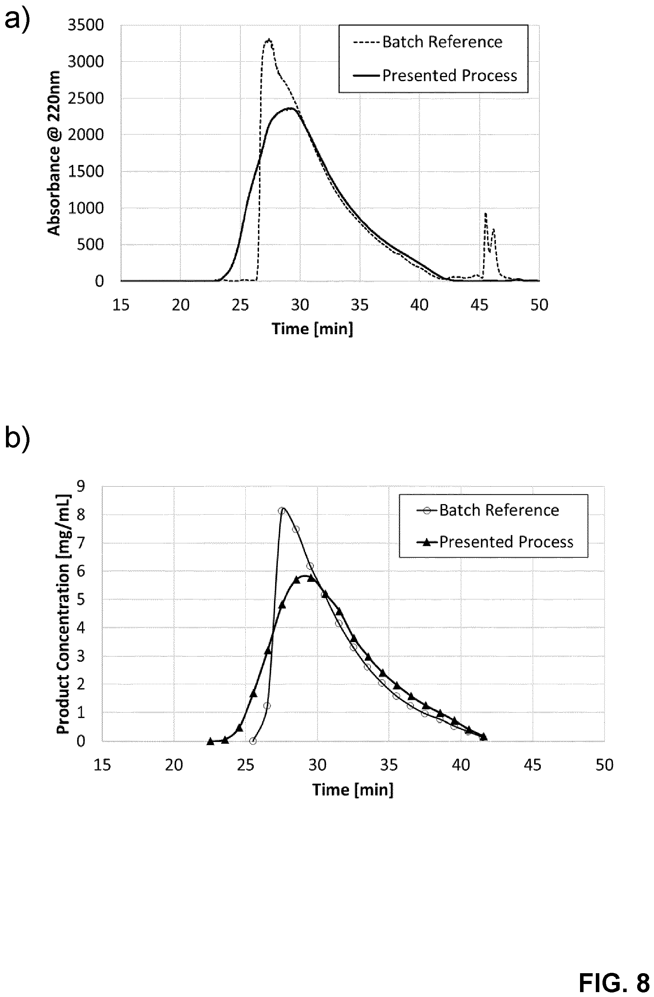

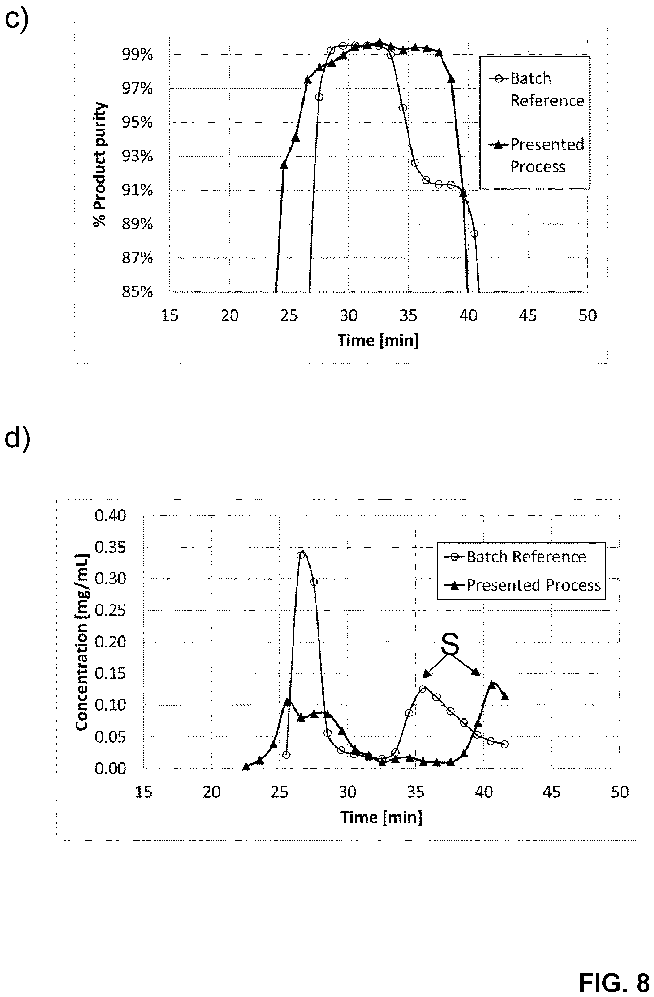

- Fig. 8: a) shows an overlay of the UV profiles of the Shutdown Phase of the presented process and the single column reference run, b) shows an overlay of the product concentration values determined by offline HPLC analysis of the Shutdown Phase of the presented process and the single column reference run, c) shows an overlay of the product purity values determined by offline HPLC analysis of the Shutdown Phase of the presented process and the single column reference run, and d) shows an overlay of the impurity content values determined by offline HPLC analysis of the Shutdown Phase of the presented process and the single column reference run. The strongly adsorbing impurity S main peaks are indicated with arrows in d);

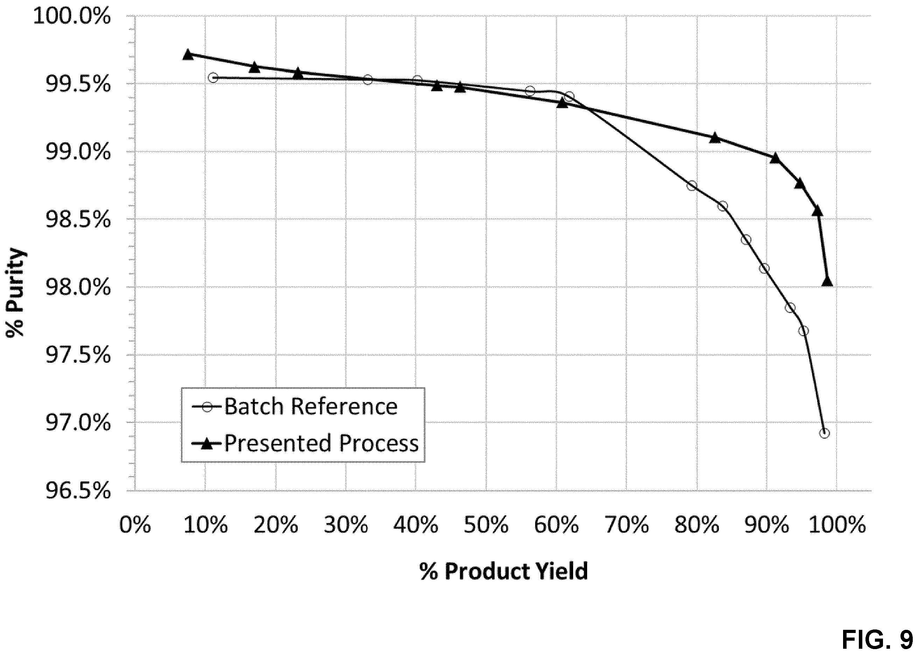

- Fig. 9: shows an overlay of the purity / yield curves of the presented process and the single column reference run;

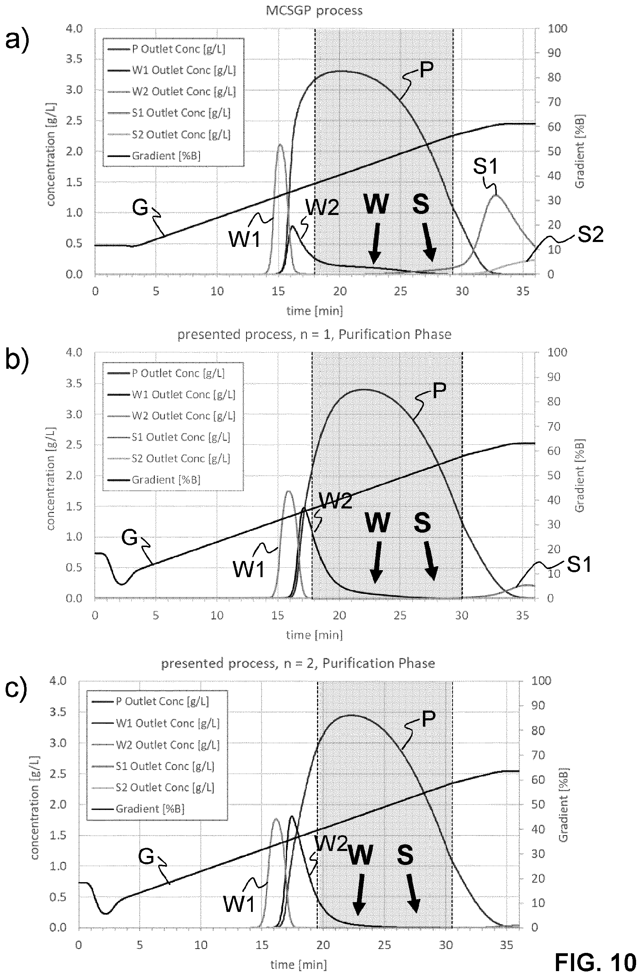

- Fig. 10: in a) shows the internal chromatogram of a regular MCSGP process recorded at the column outlet of the upstream column throughout the phases IC1, B1, IC2 and B2; in b) shows the internal chromatogram of the Purification Phase of the presented process for n =1, recorded at the column outlet of the upstream column throughout the steps IC1, B1, IC2 and B2; and in c) shows the internal chromatogram of the Purification Phase of the presented process for n =2, recorded at the column outlet of the upstream column throughout the steps IC1, B1, IC2 and B2; the rectangular shaped areas indicate the product collection interval and the arrows denoted with "W" point at the position of weakly adsorbing impurities and the arrow denoted with "S" point at the position of strongly adsorbing impurities.

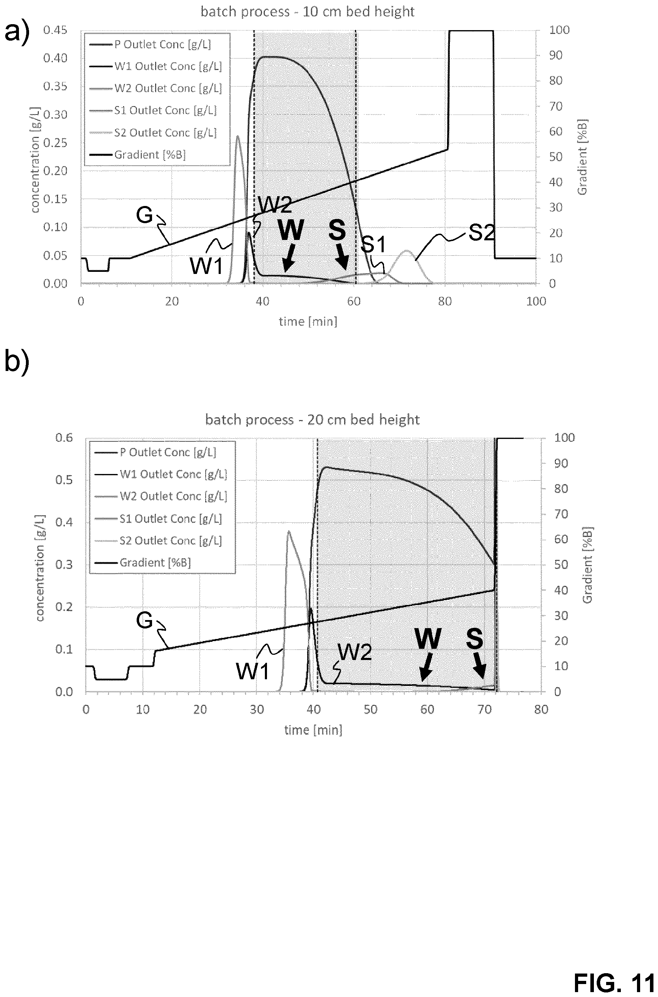

- Fig. 11: in a) shows the chromatogram of the batch reference process with 10 cm bed height; and in b) shows the chromatogram of the batch reference process with 20 cm bed height. The rectangular shaped areas indicate the product collection interval and the arrows denoted with "W" point at the position of weakly adsorbing impurities and the arrow denoted with "S" point at the position of strongly adsorbing impurities.

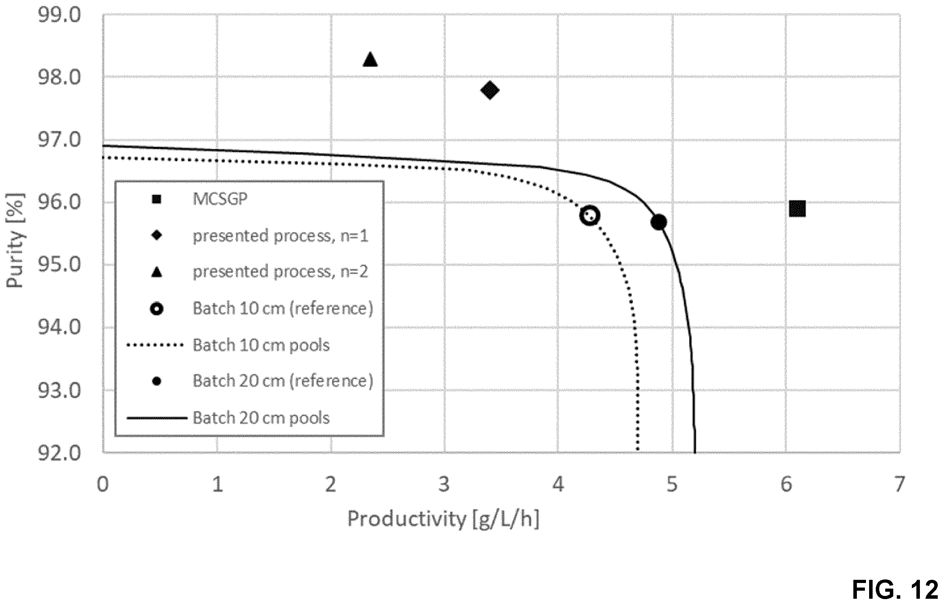

- Fig. 12: shows product pool purity vs. productivity for 1. the presented process with n=1 sequences in the Recycling Phase, 2. the presented process with n=2 sequences in the Recycling Phase, the MCSGP process, a single column batch reference process with 10 cm bed height and a single column batch reference process with 20 cm bed height. For the batch processes, the figure also shows the purity/productivity curve whose points correspond to product pools of various sizes.

### DESCRIPTION OF PREFERRED EMBODIMENTS

### Example 1: Purification of Angiotensin II

A feed solution containing 2.1 g/L Angiotensin II from chemical synthesis was prepared in solvent A (5% acetonitrile (ACN) + 0.1% trifluoroacetic acid (TFA) in water). For the chromatographic runs, solvent A and solvent B (50% ACN + 0.1% TFA in water) were used. The feed purity was %W=4.76, %P=91.67, %S=3.57 (in each case area % based on analytical HPLC).

Two columns of 2.5 mL column volume (0.46 cm inner diameter x 15 cm bed height) were used to run the presented process. For reference, single column runs were performed using the same materials.

The presented process was run on a Contichrom CUBE system (ChromaCon) using the parameters as given in Table 1.

To investigate the effects of the Recycling Phase, a Shutdown Phase was run directly after the Recycling Phase and the product peak was fractionated in a non-cyclic non-continuous process. A comparison of the fractionated shutdown and a fractionation of the single column reference run shows that with the presented process the product peak was wider, as visible both in the UV detector signal recorded at the column outlet (Figure 8a) and confirmed by offline analysis using analytical HPLC (Figure 8b). Yet the achieved purity, as determined by analytical HPLC, was higher (Figure 8c) over a significantly wider range of fractions in the presented process. Offline HPLC analysis confirmed that the strongly adsorbing impurities S have been significantly retarded in the presented process, allowing for inclusion of larger number of fractions in the product pool **(****Figure 8d****).**

Plotting the purity-yield curves of the batch reference process and the presented process **(*****Figure 9*****)** shows that with the same initial load of 16 g/L, the presented process offers higher yields for given purities. The individual points of the yield-purity curves represent pools of various sizes obtained by grouping fractions.

**Table 1 - Operating parameters of the Contichrom CUBE system to examine the effect of the Recycling Phase of the presented process.**

| **Step** | | **Startup** | **Recycle** | **Shutdown** |
|---|---|---|---|---|
| ***Feed concentration*** | | | | |
| Load | [g/L] | 16.8 | - | - |

| ***Modifier concentration*** | | | | |
|---|---|---|---|---|
| Equilibration before load | [%B] | 10% | - | 10% |
| Load | [%B] | - | - | - |
| Wash after load | [%B] | 10% | - | - |
| Gradient Start | [%B] | - | 10% | 10% |
| Gradient End | [%B] | - | 50% | 50% |
| Clean (Strip) | [%B] | - | 100% | 100% |

| ***Flow rates*** | | | | |
|---|---|---|---|---|
| Equilibration before load | [cm/h] | 600 | - | 500 |
| Loading | [cm/h] | 300 | - | - |
| Wash after load | [cm/h] | 300 | - | - |
| Gradient flow rate | [cm/h] | - | 217 | 300 |
| In-line dilution flow rate | [cm/h] | | 434 | - |
| Clean (Strip) | [cm/h] | - | 600 | 300 |
| Re-equilibration | [cm/h] | - | 600 | 500 |

| ***Flow volume*** | | | | |
|---|---|---|---|---|
| Equilibration before load | [CV] | 3 | - | 3 |
| Load | [CV] | 4 | - | - |
| Wash after load | [CV] | 2 | - | - |
| Gradient duration | [CV] | 10 | 10 | 10 |
| Clean (Strip) | [CV] | 2 | 2 | 2 |
| Re-equilibration | [CV] | 4 | 4 | 4 |

| | | | | |
|---|---|---|---|---|
| CV: relative volume per column volume; %B proportion of solvent B, complement to 100% given by solvent A. | | | | |

### Example 2: Numerical simulation of Oligonucleotide Purification

The presented process was simulated for the purification of an oligonucleotide by anion exchange chromatography. The feed mixture for simulations consisted of a 20mer dsDNA oligonucleotide and W and S impurities: P = 2.865 g/L (87.2% pure), W1 = 0.13 g/L, W2 = 0.23 g/L, S1 = 0.08 g/L, S2 = 0.13 g/L. The gradient is illustrated with G in Figs. 10 and 11. Two columns of 0.5 cm inner diameter and 10 cm bed height, packed with YMC SmartSep Q30 were used. For the simulations, a mechanistic model based on a bi-Langmuir adsorption isotherm was employed and calibrated using a set of single column gradient experiments with various gradient slopes and loads.

**Figure 10a** shows the internal chromatograms of a regular MCSGP process according to EP-A-1 877 769 recorded at the column outlet of the upstream column throughout the phases IC1, B1, IC2 and B2.

Likewise, **Figure 10b** and **Figure 10c** show the internal chromatograms of the Purification Phases for n=1 (Figure 10b) and n=2 (Figure 10c), i.e. for one or two recycling phases, respectively, recorded at the column outlet of the upstream column throughout the respective phases IC1, B1, IC2 and B2. The rectangular shaded areas in Figures 10 a-c indicate the product collection intervals. Within the product collection window it can be seen that with increasing repetitions of the Recycling Phase, ranging from n =0 (regular MCSGP process, Fig. 10a), over n =1 (one repetition of the Recycling Phase, Fig. 10b) to n =2 (two repetitions of the Recycling Phase, Fig. 10c), the product collected in the product elution window becomes much purer. This is a consequence of the better removal of "W" and "S" impurities that are indicated by arrows in Figures 10 a-c.

In the table the operating parameters of the simulated system to examine are given.

**Table 2 - Parameters of the system to examine the effect of the presented process ("n.a." - not applicable).**

| **Step** | | **Presented Process n=1** | **Presented Process n=2** | **MCSGP** |
|---|---|---|---|---|
| ***Startup Phase B-SU-F*** | | | | |
| Phase Duration | [min] | 31 | 31 | n.a. |
| Load Duration | [min] | 11 | 11 | n.a. |
| Load | [g/L] | 9.3 | 9.3 | n.a. |
| Feed flow rate | [cm/h] | 150 | 150 | n.a. |
| Wash flow rate | [cm/h] | 150 | 150 | n.a. |
| Wash concentration (constant) | [%B] | 0 | 0 | n.a. |
| | | | | |

| ***Startup Phase B-SU-W*** | | | | |
|---|---|---|---|---|
| Duration | [min] | 17 | 17 | n.a. |
| Gradient flow rate | [cm/h] | 150 | 150 | n.a. |
| Gradient starting concentration | [%B] | 10.5 | 10.5 | n.a. |
| Gradient final concentration | [%B] | 40.1 | 40.1 | n.a. |
| | | | | |

| ***Interconnected Phase IC-R*** | | | | |
|---|---|---|---|---|
| Duration | [min] | 30 | 30 | n.a. |
| Gradient flow rate | [cm/h] | 107 | 107 | n.a. |
| In-line dilution flow rate | [cm/h] | 400 | 400 | n.a. |
| Gradient starting concentration | [%B] | 30 | 30 | n.a. |
| Gradient final concentration | [%B] | 60 | 60 | n.a. |
| | | | | |

| ***Batch Phase B-R*** | | | | |
|---|---|---|---|---|
| Duration | [min] | 0 | 0 | n.a. |
| Cleaning flow rate (S-elution) | [cm/h] | 0 | 0 | n.a. |
| Gradient flow rate (W-elution) | [cm/h] | 0 | 0 | n.a. |
| | | | | |

| ***Interconnected Phase IC1*** | | | | |
|---|---|---|---|---|
| Duration | [min] | 2.2 | 2.2 | 2.2 |
| Gradient flow rate | [cm/h] | 150 | 150 | 150 |
| In-line dilution flow rate | [cm/h] | 409 | 409 | 409 |
| Gradient starting concentration | [%B] | 40.1 | 41.2 | 38.0 |
| Gradient final concentration | [%B] | 44.0 | 44.8 | 41.2 |
| | | | | |

| ***Batch Phase B1*** | | | | |
|---|---|---|---|---|
| Duration | [min] | 11.0 | 11.0 | 11.0 |
| Gradient flow rate (P-elution) | [cm/h] | 150 | 150 | 150 |
| Gradient starting concentration | [%B] | 44.0 | 44.8 | 41.2 |
| Gradient final concentration | [%B] | 63.2 | 64.0 | 61.1 |
| Feed flow rate | [cm/h] | 150 | 150 | 150 |
| | | | | |

| ***Interconnected Phase IC2*** | | | | |
|---|---|---|---|---|
| Duration | [min] | 8.20 | 8.20 | 8.20 |
| Gradient flow rate | [cm/h] | 107 | 107 | 107 |
| In-line dilution flow rate | [cm/h] | 400 | 400 | 400 |
| Gradient starting concentration | [%B] | 63.2 | 64.0 | 61.1 |
| Gradient final concentration | [%B] | 63.2 | 64.0 | 61.1 |
| | | | | |

| ***Batch Phase B2*** | | | | |
|---|---|---|---|---|
| Duration | [min] | 17.0 | 17.6 | 15.7 |
| Cleaning flow rate (S-elution) | [cm/h] | 562 | 562 | 562 |
| Gradient flow rate (W-elution) | [cm/h] | 150 | 150 | 150 |
| Gradient starting concentration (S-elu.) | [%B] | 100 | 100 | 100 |
| Gradient final concentration (S-elu.) | [%B] | 100 | 100 | 100 |
| Gradient starting concentration (W-elu.) | [%B] | 6 | 6 | 6 |
| Gradient final concentration (W-elu.) | [%B] | 40.1 | 41.2 | 38.0 |
| | | | | |

| ***Shutdown Phase I (IC-R)*** | | | | |
|---|---|---|---|---|
| Duration | [min] | 30 | 30 | n.a. |
| Gradient flow rate | [cm/h] | 107 | 107 | n.a. |
| In-line dilution flow rate | [cm/h] | 400 | 400 | n.a. |
| Gradient starting concentration | [%B] | 30 | 30 | n.a. |
| Gradient final concentration | [%B] | 60 | 60 | n.a. |
| | | | | |

| ***Shutdown Phase I (IC-B)*** | | | | |
|---|---|---|---|---|
| Duration | [min] | 0 | 0 | n.a. |
| Cleaning flow rate (S-elution) | [cm/h] | 0 | 0 | n.a. |
| Gradient flow rate (W-elution) | [cm/h] | 0 | 0 | n.a. |
| | | | | |

| ***Shutdown Phase II (IC1-SD)*** | | | | |
|---|---|---|---|---|
| Duration | [min] | 2.2 | 2.2 | n.a. |
| Gradient flow rate | [cm/h] | 150 | 150 | n.a. |
| In-line dilution flow rate | [cm/h] | 409 | 409 | n.a. |
| Gradient starting concentration | [%B] | 40.1 | 40.1 | n.a. |
| Gradient final concentration | [%B] | 44.0 | 44.0 | n.a. |
| | | | | |

| ***Shutdown Phase II (B1-SD)*** | | | | |
|---|---|---|---|---|
| Duration | [min] | 11.0 | 11.0 | n.a. |
| Gradient flow rate (P-elution) | [cm/h] | 150 | 150 | n.a. |
| Gradient starting concentration | [%B] | 44.0 | 44.0 | n.a. |
| Gradient final concentration | [%B] | 63.2 | 63.2 | n.a. |
| Feed flow rate | [cm/h] | 0 | 0 | n.a. |
| | | | | |

| ***Shutdown Phase II (IC2-SD)*** | | | | |
|---|---|---|---|---|
| Duration | [min] | 8.20 | 8.20 | n.a. |
| Gradient flow rate | [cm/h] | 107 | 107 | n.a. |
| In-line dilution flow rate | [cm/h] | 400 | 400 | n.a. |
| Gradient starting concentration | [%B] | 63.2 | 63.2 | n.a. |
| Gradient final concentration | [%B] | 63.2 | 63.2 | n.a. |
| | | | | |

| ***Shutdown Phase II (B2-SD)*** | | | | |
|---|---|---|---|---|
| Duration | [min] | 17.0 | 17.0 | n.a. |
| Cleaning flow rate (S-elution) | [cm/h] | 562 | 562 | n.a. |
| Gradient flow rate (W-elution) | [cm/h] | 150 | 150 | n.a. |
| Gradient starting concentration | [%B] | 10.5 | 10.5 | n.a. |
| Gradient final concentration | [%B] | 40.1 | 40.1 | n.a. |
| | | | | |
| ***Shutdown Phase II (B1-SD-P)*** | | | | |
| Duration | [min] | 11.0 | 11.0 | n.a. |
| Gradient flow rate (P-elution) | [cm/h] | 150 | 150 | n.a. |
| Gradient starting concentration | [%B] | 44.0 | 44.0 | n.a. |
| Gradient final concentration | [%B] | 63.2 | 63.2 | n.a. |
| | | | | |

| ***Shutdown Phase II (B1-SD-S)*** | | | | |
|---|---|---|---|---|
| Duration | [min] | 17.0 | 17.0 | n.a. |
| Cleaning flow rate (S-elution) | [cm/h] | 562 | 562 | n.a. |
| Gradient starting concentration (S-elu.) | [%B] | 100 | 100 | n.a. |
| Gradient final concentration (S-elu.) | [%B] | 100 | 100 | n.a. |

For comparison, **Figures 11** a and b show the chromatograms of batch reference processes with 10cm and 20 cm bed height, respectively. As in Figures 10a-c, the rectangular shaped areas indicate the product collection intervals and the arrows denoted with "W" point at the position of relevant overlapping weakly adsorbing impurities and the arrow denoted with "S" point at the position of relevant overlapping strongly adsorbing impurities. It can be seen that the product pool contains much lower amounts of impurities W and S the more recycling phases are used.

The results were compared in terms of product pool purity vs. productivity and are shown in **Figure 12****.** In addition to the twin-column processes, single column reference processes were simulated. For the single column reference processes, product pools of varying size were extracted from the simulated chromatograms, corresponding to pools with different product and impurity content, and therefore corresponding to different productivities and purities.

It can be seen that much higher purities can be obtained with the presented process (> 97.5% purity, both for n=1 and n=2) than with the reference batch processes (< 97.0% purity) at comparable productivity. In comparison to a "regular" MCSGP process according to EP-A-1 877 769, the presented process achieves higher purity (MCSGP purity < 96.0% purity) but a somewhat lower productivity. Moreover **Figure 12** confirms that by increasing the number n of sequences of the Recycling Phase, product purity can be increased further (Purity = 97.8% for n=1 vs. Purity = 98.4% for n=2), however this comes at the cost of productivity (productivity = 3.4 g/L/h for n=1 vs. productivity = 2.3 g/L/h for n=2).

### LIST OF REFERENCE SIGNS

| | | | |
|---|---|---|---|
| IC1 | first interconnected step of the Purification Phase | B1 | first disconnected step (batch step) of the Purification Phase |
| IC2 | second interconnected step of the Purification Phase | B2 | |
| | | | second disconnected step (batch step) of the Purification Phase |
| IC-R | interconnected step of the Recycling Phase or of the Shutdown Phase I | B-R | |
| | | | disconnected step (batch step) of the Recycling Phase or of the Shutdown Phase I |
| IC1-SD | first interconnected step of the Shutdown Phase II | | |
| IC2-SD | second interconnected step of the Shutdown Phase II | B1-SD | first disconnected step (batch step) of the Shutdown Phase |
| | II | t_{IC2} | duration of phase IC2 |
| B2-SD | second disconnected step (batch step) of the Shutdown Phase II | t_{IC-R} | duration of phase IC-R |
| | | t_{B1} | duration of step B1 |
| | | t_{B2} | duration of step B2 |
| B-SU-F | disconnected step (Feeding step) of Startup phase | t_{B-R} | duration of step B-R |
| | | t_{B-SU-F} | duration of step B-SU-F |
| B-SU-W | disconnected step (W elution step) of Startup phase | t_{B-SU-W} | duration of step B-SU-W |
| | | t_{S-SD-P} | duration of step B-SD-P |
| B-SD-P | disconnected step (Product elution step) of Shutdown Phase | t_{B-SD-S} | duration of step B-SD-S |
| | | P | Product |
| | | F | Feed mixture containing W, P and S |
| B-SD-S | disconnected step (S elution step) of Shutdown phase | | |
| | | W | Weakly adsorbing impurities |
| | | S | Strongly adsorbing impurities |
| t_{IC1} | duration of phase IC1 | | |

## Claims

1. A cyclic chromatographic purification method for the isolation of a product (P) from a feed mixture (F) consisting of the product (P) and at least two further components representing weakly adsorbing impurities (W) and strongly adsorbing impurities (S),
the method using only two chromatographic adsorber sections as chromatographic stationary phase, wherein a first adsorber section (1) has a first adsorber section inlet and a first adsorber section outlet, and a second adsorber section (2) has a second adsorber section inlet and a second adsorber section outlet,
wherein the method comprises at least one base sequence having at least one Recycling Phase followed by only one Purification Phase, wherein preferably base sequences repeat in a cyclic manner at least twice,
and wherein said Recycling Phase consists of at least one recycling sequence comprising the following steps:
a. an interconnected recycling step (IC-R), in which the adsorber sections are interconnected, and in which an upstream adsorber section outlet is connected to a downstream adsorber section inlet during a recycling interconnected timespan (t_{IC-R}),
wherein the upstream adsorber section is loaded via the upstream adsorber section inlet with eluent and wherein a fraction containing the weakly adsorbing impurities (W) at least as far as overlapping with product (P), the product (P), and the strongly adsorbing impurities (S) at least as far as overlapping with product (P) is eluted from the upstream adsorber section into the downstream adsorber section and wherein the stream exiting the upstream adsorber outlet is diluted inline before entering the downstream adsorber inlet;
b. an optional batch recycling step (B-R), wherein the during a recycling batch timespan (t_{B-R}) said adsorber sections are disconnected and wherein the previously upstream adsorber section of preceding step a. is cleaned to remove the strongly adsorbing impurities (S) and regenerated and wherein eluent is loaded to the inlet of the previously downstream adsorber section of step a. to elute weakly adsorbing impurities (W) as far as not overlapping with product (P);
wherein after each recycling sequence of steps a. and b., the adsorber sections are switched in order, wherein the number of recycling sequences is ≥ 1,
and wherein each Recycling Phase is followed by only one Purification Phase, wherein the Purification Phase comprises the following steps in order, wherein the upstream adsorber section of the last interconnected recycling step of the preceding Recycling Phase takes the function of the downstream adsorber section and the downstream adsorber section of the last interconnected recycling step of the preceding Recycling Phase takes the function of the upstream adsorber section:
c. a first interconnected purification step (IC1), in which the adsorber sections are interconnected, wherein the upstream adsorber section outlet is connected to the downstream adsorber section inlet during a first interconnected purification timespan (t_{IC1}),
wherein the upstream adsorber is loaded via the upstream adsorber inlet with eluent and wherein a product fraction containing weakly adsorbing impurities (W) and Product (P) in overlap is eluted from the upstream adsorber into the downstream adsorber section and wherein the stream exiting the upstream adsorber section outlet is diluted inline before entering the downstream adsorber section inlet
d. a first batch purification step (B1), wherein during a first batch purification timespan (t_{B1}) said adsorber sections are disconnected and wherein product (P) is eluted from the previous upstream adsorber section and wherein feed mixture (F) is supplied to the previous downstream adsorber section inlet
e. a second interconnected purification step (IC2), wherein the upstream adsorber section outlet is connected to the downstream adsorber section inlet during a second interconnected purification timespan (t_{IC2}),
wherein the upstream adsorber section is loaded via the upstream adsorber section inlet with eluent and wherein a product fraction containing Product (P) and strongly adsorbing impurities (S) in overlap is eluted from the upstream adsorber into the downstream adsorber and
wherein the stream exiting the upstream adsorber section outlet is diluted inline before entering the downstream adsorber section inlet
f. a second batch purification step (B2), wherein during a second purification batch timespan (t_{B2}) said adsorbers are disconnected and wherein the previous upstream adsorber is cleaned and regenerated and wherein eluent is loaded to the previous downstream adsorber inlet.

2. A method according to claim 1, wherein eluent gradients, preferably linear eluent gradients, are used in at least one or all of the phases, wherein preferably a higher gradient slope in the Recycling Phase steps (IC-R and/or B-R) is selected than for the Purification Phase steps (IC1, B1, IC2, and/or B2).

3. Method according to any of the preceding claims, wherein in interconnected steps of the method the upstream section inlet is loaded via the upstream adsorber inlet with eluent with a gradient in the form of a temporally changing modifier concentration and wherein the stream exiting the upstream adsorber section outlet is diluted inline before entering the downstream adsorber section inlet with eluent without modifier or with a different modifier concentration than at the inlet of the upstream adsorber section,

4. Method according to any of the preceding claims, wherein in batch steps of the method without purified product elution the previous upstream adsorber is cleaned, preferably with eluent with a higher modifier concentration than at the end of the preceding interconnected recycling step or with a different modifier or with a cleaning solution, and regenerated and wherein eluent with a gradient in the form of a temporally changing modifier concentration is loaded to the previous downstream adsorber inlet
and/or wherein in batch steps of the method with purified product elution the previous upstream adsorber is loaded via the upstream adsorber inlet with eluent with a gradient in the form of a temporally changing modifier concentration.

5. Method according to any of the preceding claims, wherein in said Recycling Phase in said interconnected recycling step (IC-R), the upstream adsorber section is loaded via the upstream adsorber section inlet with eluent with a gradient in the form of a temporally changing modifier concentration, and wherein the stream exiting the upstream adsorber outlet is diluted inline before entering the downstream adsorber inlet with eluent without modifier or with a different modifier concentration than at the inlet of the upstream adsorber section;
and/or wherein in the optional batch recycling step (B-R), the previously upstream adsorber section of preceding step a. is cleaned, preferably with eluent with a higher modifier concentration than at the end of the preceding interconnected recycling step or with a different modifier or with a cleaning solution, and regenerated and wherein eluent with a gradient in the form of a temporally changing modifier concentration is loaded to the inlet of the previously downstream adsorber section of step a. to elute weakly adsorbing impurities (W) as far as not overlapping with product (P).

6. Method according to any of the preceding claims, wherein in said Purification Phase in said first interconnected purification step (IC1) the upstream adsorber section is loaded via the upstream adsorber inlet with eluent with a gradient in the form of a temporally changing modifier concentration and wherein the stream exiting the upstream adsorber section outlet is diluted inline before entering the downstream adsorber section inlet with eluent without modifier or with a different modifier concentration than at the inlet of the upstream adsorber section;
and/or wherein in said first batch purification step (B1) product (P) is eluted from the previous upstream adsorber section by loading it via its inlet with eluent with a gradient in the form of a temporally changing modifier concentration;
and/or wherein in said second interconnected purification step (IC2) said upstream adsorber section inlet is loaded via the upstream adsorber inlet with eluent with a gradient in the form of a temporally changing modifier concentration and wherein the stream exiting the upstream adsorber section outlet is diluted inline before entering the downstream adsorber section inlet with eluent without modifier or with a different modifier concentration than at the inlet of the upstream adsorber section;
and/or wherein in said second batch purification step (B2) the previous upstream adsorber is cleaned, preferably with eluent with a higher modifier concentration than at the end of the preceding interconnected recycling step or with a different modifier or with a cleaning solution, and regenerated and wherein eluent with a gradient in the form of a temporally changing modifier concentration is loaded to the previous downstream adsorber inlet.

7. Method according to any of the preceding claims 2-6, wherein the modifier is selected from the group consisting of an organic or inorganic solvent or mixture thereof different from a base solvent or mixture thereof of the eluent, an electrolyte in such an organic or inorganic solvent (or a mixture thereof), preferably selected from a dissolved salt or a pH, or a combination thereof, wherein
preferably said base solvent is water or a mixture of water with at least one organic solvent or water in a mixture with one or more salts and/or organic solvents one or both in a minor proportion compared with water, and wherein
further preferably said modifier is an organic solvent or a mixture of water with at least one organic solvent having a higher concentration of said at least one organic solvent than in the base solvent, water or a mixture of water with at least one organic solvent with a different salt or H⁺ concentration than the base solvent.

8. Method according to any of the preceding claims, wherein, preferably linear, eluent gradients are used in at least one or all of the phases with a gradient in the form of a temporally changing modifier concentration increase.

9. Method according to any of the preceding claims, wherein before carrying out the first interconnected recycling step of the first Recycling Phase, a Start-up Phase is carried out, in which
during a first batch start-up timespan (t_{B-SU-F}) said adsorbers (1,2) are disconnected
and feed mixture (F) is supplied to inlet of the adsorber section (1) to become the upstream adsorber section of the first interconnected recycling step of the first Recycling Phase,
while the adsorber section (2) to become the downstream adsorber section of the first interconnected recycling step of the first Recycling Phase is either being equilibrated or already equilibrated and inactive,
and wherein preferably subsequently, during a second batch start-up timespan (t_{B'-SU-W}) said adsorbers (1,2) are disconnected and weakly adsorbing impurity (W) is eluted from the adsorber section (1) having been supplied with feed mixture (F) in the preceding first batch start-up step while the other adsorber (2) is either being equilibrated or already equilibrated and inactive.

10. Method according to claim 9, wherein after said first batch start-up timespan (t_{B-SU-F}) and before said second batch start-up timespan (t_{B-SU-W}) eluent is supplied to inlet of the adsorber section to become the upstream adsorber section of the first interconnected recycling step of the first Recycling Phase, and wherein said eluent is without modifier or with a modifier concentration essentially corresponding to the starting modifier concentration applied during said first batch start-up timespan or in the absence of a second batch start-up timespan of the first interconnected recycling step;
and/or wherein during said second batch start-up timespan the inlet of the adsorber section (1) having been supplied with feed mixture (F) is loaded with eluent with a gradient in the form of a temporally changing modifier concentration.

11. Method according to any of the preceding claims, wherein after termination of at least one base sequence, preferably more than one base sequences, a Shut-down Phase is carried out,
wherein the Shutdown Phase comprises the following steps in order:
a'. an interconnected shutdown recycling step (IC'-R), in which the upstream adsorber section of the preceding and last second interconnected purification step (IC2) takes the downstream position and the other adsorber section takes the upstream position, in which the adsorber sections are interconnected, and in which the upstream adsorber section outlet is connected to the downstream adsorber section inlet during a shutdown recycling interconnected timespan (t_{IC'-R-SD}),
wherein the upstream adsorber section is loaded via the upstream adsorber section inlet with eluent and wherein a fraction containing the weakly adsorbing impurities (W) at least as far as overlapping with product (P), the product (P), and the strongly adsorbing impurities (S) at least as far as overlapping with product (P) is eluted from the upstream adsorber section into the downstream adsorber section and wherein the stream exiting the upstream adsorber outlet is diluted inline before entering the downstream adsorber inlet;
b'. an optional batch shutdown recycling step (B'-R), in which the adsorber sections are disconnected, wherein during a shutdown recycling batch timespan (t_{B'-R}) said adsorber sections are disconnected and
wherein the previously upstream adsorber section of preceding step a'. is cleaned and regenerated and wherein eluent is loaded to the inlet of the previously downstream adsorber section of step a to elute weakly adsorbing impurities (W) in as far as not overlapping with product (P);
followed by the following steps in order, wherein the upstream adsorber section of the interconnected shutdown recycling step takes the function of the downstream adsorber section and the downstream adsorber section of the interconnected shutdown recycling step takes the function of the upstream adsorber section:
c'. a first interconnected shutdown purification step (IC1-SD), in which the adsorber sections are interconnected, wherein the upstream adsorber section outlet is connected to the downstream adsorber section inlet during a first interconnected shutdown purification timespan (t_{IC1'}),
wherein the upstream adsorber is loaded via the upstream adsorber inlet with eluent and wherein a product fraction containing weakly adsorbing impurities (W) and Product (P) in overlap is eluted from the upstream adsorber into the downstream adsorber section and wherein the stream exiting the upstream adsorber section outlet is diluted inline before entering the downstream adsorber section inlet
d'. a first batch shutdown purification step (B1-SD), wherein during a first batch purification timespan (t_{B1'}) said adsorber sections are disconnected and wherein product (P) is eluted from the previous upstream adsorber section and wherein the previous downstream adsorber section is either idle or eluent is supplied to the previous downstream adsorber section inlet
e'. a second interconnected shutdown purification step (IC2-SD), wherein the upstream adsorber section outlet is connected to the downstream adsorber section inlet during a second interconnected shutdown purification timespan (t_{IC2'}),
wherein the upstream adsorber section is loaded via the upstream adsorber section inlet with eluent and wherein a product fraction containing Product (P) and strongly adsorbing impurities (S) in overlap is eluted from the upstream adsorber into the downstream adsorber and
wherein the stream exiting the upstream adsorber section outlet is diluted inline before entering the downstream adsorber section inlet
f'. a second batch shutdown purification step (B2-SD), wherein during a second purification batch timespan (t_{B2'}) said adsorbers are disconnected and wherein the previous upstream adsorber is cleaned and regenerated and wherein eluent is loaded to the previous downstream adsorber inlet
followed by
g'. a first final shutdown batch step (B-SD-P), wherein during a first final shutdown batch timespan (t_{B-SD-P}) said first and second adsorbers are disconnected and wherein product (P) is eluted from the previously downstream adsorber and wherein the other adsorber is idle or regenerated
h'. optionally but preferably a second final shutdown batch step (B-SD-S), wherein during a second final shutdown batch timespan (t_{B-SD-S}) said first and second adsorbers are disconnected and strongly adsorbing impurity S is eluted from the previously downstream adsorber and wherein the other adsorber is idle or regenerated.

12. Method according to claim 11, wherein in said interconnected shutdown recycling step (IC'-R), the upstream adsorber section of the preceding and last second interconnected purification step (IC2) is loaded via the upstream adsorber section inlet with eluent of constant composition or with a gradient in the form of a temporally changing modifier concentration wherein the stream exiting the upstream adsorber outlet is diluted inline before entering the downstream adsorber inlet with eluent without modifier or with a different modifier concentration than at the inlet of the upstream adsorber section;
and/or wherein in said batch shutdown recycling step (B'-R) the previously upstream adsorber section of preceding step a. is cleaned and regenerated and wherein eluent with a gradient in the form of a temporally changing modifier concentration is loaded to the inlet of the previously downstream adsorber section of step a'. to elute weakly adsorbing impurities (W) in as far as not overlapping with product (P);
and/or wherein in said first interconnected shutdown purification step (IC1-SD) the upstream adsorber is loaded via the upstream adsorber inlet with eluent with a gradient in the form of a temporally changing modifier concentration and wherein the stream exiting the upstream adsorber section outlet is diluted inline before entering the downstream adsorber section inlet with eluent without modifier or with a different modifier concentration than at the inlet of the upstream adsorber section:
and/or wherein in said first batch shutdown purification step (B1-SD) product (P) is eluted from the previous upstream adsorber section by loading via the upstream adsorber inlet with eluent with a gradient in the form of a temporally changing modifier concentration and wherein the previous downstream adsorber section is either idle or eluent is supplied to the previous downstream adsorber section inlet, preferably eluent in the form of the base solvent;
and/or wherein in said second interconnected shutdown purification step (IC2-SD) the upstream adsorber section is loaded via the upstream adsorber section inlet with eluent of constant composition or with a gradient in the form of a temporally changing modifier concentration and wherein the stream exiting the upstream adsorber section outlet is diluted inline before entering the downstream adsorber section inlet with eluent without modifier or with a different modifier concentration than at the inlet of the upstream adsorber section;
and/or wherein in said second batch shutdown purification step (B2-SD), the previous upstream adsorber is cleaned and regenerated and wherein eluent is loaded to the previous downstream adsorber inlet;
and/or wherein in said first final shutdown batch step (B-SD-P) product (P) is eluted from the previously downstream adsorber with eluent with a gradient in the form of a temporally changing modifier concentration and wherein the other adsorber is idle or regenerated;
and/or wherein in said first final second final shutdown batch step (B-SD-S), strongly adsorbing impurity S is eluted from the previously downstream adsorber and wherein the other adsorber is idle or regenerated.

13. A method according to any of the preceding claims, wherein linear gradients with different slopes and/or flow rates are used in the Recycling Phase and/or in the Purification Phase.

14. Method according to any of the preceding claims 2-13, wherein in most or in all interconnected steps of the method the upstream section inlet is loaded via the upstream adsorber inlet with eluent with a gradient in the form of a temporally changing modifier concentration and wherein the stream exiting the upstream adsorber section outlet is diluted inline before entering the downstream adsorber section inlet with eluent without modifier or with a different modifier concentration than at the inlet of the upstream adsorber section,
and wherein
in batch steps of the method without purified product elution the previous upstream adsorber is cleaned with eluent and regenerated and, if applicable, wherein eluent with a gradient in the form of a temporally changing modifier concentration is loaded to the previous downstream adsorber inlet
or in batch steps of the method with purified product elution the previous upstream adsorber is loaded via the upstream adsorber inlet with eluent with a gradient in the form of a temporally changing modifier concentration
and wherein the modifier is selected from the group consisting of a solvent or mixture thereof different from a base solvent or mixture thereof of the eluent, an electrolyte in such a solvent or mixture thereof, preferably selected from a dissolved salt or a pH, or a combination thereof, wherein preferably said base solvent is water or a mixture of water with at least one organic solvent or water in a mixture with one or more salts and/or organic solvents in a minor proportion compared with water, and wherein further preferably said modifier is an organic solvent or a mixture of water with at least one organic solvent having a higher concentration of said at least one organic solvent than the base solvent, water or a mixture of water with different salt or H⁺ concentration than the base solvent,
and wherein said the modifier concentration is increased, preferably linearly increased, in the process from a lowest concentration at the beginning of the elution (B-SU-W, B-R, B'-R, B2, B2-SD) of the weakly adsorbing impurities (W) as far as not overlapping with product (P), over the following steps of
recycling (IC-R, IC'-R, IC1, IC1-SD), except for the case of a following final shutdown batch step (B-SD-P) and
if applicable, of product elution (B1, B1-SD, B-SD-P) and, if applicable, further recycling (IC2, IC2-SD), wherein during said recycling (IC2, IC2-SD) the modifier concentration can be varied or just for the recycling (IC2, IC2-SD) be kept constant
to a highest concentration before the beginning of the elution (B-R, B'-R, B2, B2-SD, B-SD-S) of the strongly adsorbing impurities (S).

15. Use of a method according to any of the preceding claims for the purification of biomolecules, of natural or synthetic origin, preferably selected from the group consisting of nucleic acid molecules, including DNA and RNA molecules, proteins, including antibodies, peptides, carbohydrates, lipids as well as combinations and modifications as well as fragments thereof.

## Patentansprüche

1. Zyklisch-chromatographisches Reinigungsverfahren zur Isolierung eines Produkts (P) aus einer Beschickungsmischung (F), die aus dem Produkt (P) und mindestens zwei weiteren Komponenten besteht, die schwach adsorbierende Verunreinigungen (W) und stark adsorbierende Verunreinigungen (S) darstellen,
unter Verwendung von nur zwei chromatographischen Adsorberabschnitten als chromatographische stationäre Phase, wobei ein erster Adsorberabschnitt (1) einen ersten Adsorberabschnittseinlass und einen ersten Adsorberabschnittsauslass aufweist, und ein zweiter Adsorberabschnitt (2) einen zweiten Adsorberabschnittseinlass und einen zweiten Adsorberabschnittsauslass aufweist,
wobei das Verfahren mindestens eine Basissequenz mit mindestens einer Recyclingphase gefolgt von nur einer Reinigungsphase umfasst, wobei sich die Basissequenzen vorzugsweise mindestens zweimal zyklisch wiederholen,
und wobei die Recyclingphase aus mindestens einer Recyclingsequenz besteht, die die folgenden Schritte umfasst:
a. einen verbundenen recyclingschritt (IC-R), bei dem die Adsorberabschnitte miteinander verbunden sind und bei dem ein stromaufwärts gelegener Auslass des Adsorberabschnitts mit einem stromabwärts gelegenen Einlass des Adsorberabschnitts während einer verbunden Recyclingzeitspanne (t_{IC-R}) verbunden ist,
wobei der stromaufwärts gelegene Adsorberabschnitt über den Einlass des stromaufwärts gelegenen Adsorberabschnitts mit Eluent beladen wird und wobei eine Fraktion, die die schwach adsorbierenden Verunreinigungen (W), zumindest soweit sie sich mit dem Produkt (P) überschneiden, das Produkt (P) und die stark adsorbierenden Verunreinigungen (S), zumindest soweit sie sich mit dem Produkt (P) überschneiden, enthält, aus dem stromaufwärts gelegenen Adsorberabschnitt in den stromabwärts gelegenen Adsorberabschnitt eluiert wird und
wobei der aus dem stromaufwärts gelegenen Adsorberauslass austretende Strom in der Leitung verdünnt wird, bevor er in den stromabwärts gelegenen Adsorbereinlass eintritt;
b. einen optionalen Chargenrecyclingschritt (B-R), bei dem die Adsorberabschnitte während einer Recyclingchargenzeitspanne (t_{B-R}) getrennt werden und
wobei der zuvor stromaufwärts gelegene Adsorberabschnitt des vorhergehenden Schritts a. gereinigt wird, um die stark adsorbierenden Verunreinigungen (S) zu entfernen, und regeneriert wird, und wobei Elutionsmittel in den Einlass des zuvor stromabwärts gelegenen Adsorberabschnitts des Schritts a. geladen wird, um schwach adsorbierende Verunreinigungen (W) zu eluieren, soweit sie sich nicht mit dem Produkt (P) überlappen;
wobei nach jeder Recyclingsequenz der Schritte a. und b. die Adsorberabschnitte der Reihe nach geschaltet werden, wobei die Anzahl der Recyclingsequenzen ≥ 1 ist,
und wobei sich an jede Recyclingphase nur eine Reinigungsphase anschließt,
wobei die Reinigungsphase die folgenden Schritte in dieser Reihenfolge umfasst, wobei der stromaufwärts gelegene Adsorberabschnitt des letzten miteinander verbundenen Recyclingschritts der vorhergehenden Recyclingphase die Funktion des stromabwärts gelegenen Adsorberabschnitts übernimmt und der stromabwärts gelegene Adsorberabschnitt des letzten miteinander verbundenen Recyclingschritts der vorhergehenden Recyclingphase die Funktion des stromaufwärts gelegenen Adsorberabschnitts übernimmt:
c. einen ersten verbundenen Reinigungsschritt (IC1), in dem die Adsorberabschnitte miteinander verbunden sind, wobei der stromaufwärts gelegene Auslass des Adsorberabschnitts mit dem stromabwärts gelegenen Einlass des Adsorberabschnitts während einer ersten verbundenen Reinigungszeitspanne (t_{IC1}) verbunden ist, wobei der stromaufwärts gelegene Adsorber über den stromaufwärts gelegenen Adsorbereinlass mit Eluent beladen wird und wobei eine Produktfraktion, die schwach adsorbierende Verunreinigungen (W) und Produkt (P) in Überlappung enthält, aus dem stromaufwärts gelegenen Adsorber in den stromabwärts gelegenen Adsorberabschnitt eluiert wird und
wobei der aus dem Auslass des stromaufwärts gelegenen Adsorberabschnitts austretende Strom in der Leitung verdünnt wird, bevor er in den Einlass des stromabwärts gelegenen Adsorberabschnitts eintritt
d. einen ersten Batch-Reinigungsschritt (B1), bei dem während einer ersten Batch-Reinigungszeitspanne (t_{B1}) die Adsorberabschnitte getrennt werden und bei dem das Produkt (P) aus dem vorhergehenden stromaufwärts gelegenen Adsorberabschnitt eluiert wird und bei dem das Beschickungsgemisch (F) dem vorhergehenden stromabwärts gelegenen Adsorberabschnittseinlass zugeführt wird
e. einen zweiten verbundenen Reinigungsschritt (IC2), wobei der Auslass des stromaufwärts gelegenen Adsorberabschnitts mit dem Einlass des stromabwärts gelegenen Adsorberabschnitts während einer zweiten verbundenen Reinigungszeitspanne (t_{IC2}) verbunden ist,
wobei der stromaufwärts gelegene Adsorberabschnitt über den Einlass des stromaufwärts gelegenen Adsorberabschnitts mit Eluent beladen wird und wobei eine Produktfraktion, die Produkt (P) und stark adsorbierende Verunreinigungen (S) in Überlappung enthält, aus dem stromaufwärts gelegenen Adsorber in den stromabwärts gelegenen Adsorber eluiert wird und
wobei der aus dem Auslass des stromaufwärts gelegenen Adsorberabschnitts austretende Strom in der Leitung verdünnt wird, bevor er in den Einlass des stromabwärts gelegenen Adsorberabschnitts eintritt
f. einen zweiten Batch-Reinigungsschritt (B2), bei dem während einer zweiten Reinigungs-Batch-Zeitspanne (t_{B2}) die Adsorber getrennt werden und bei dem der vorherige stromaufwärts gelegene Adsorber gereinigt und regeneriert wird und bei dem Eluent in den vorherigen stromabwärts gelegenen Adsorbereinlass geladen wird.

2. Verfahren nach Anspruch 1, wobei Eluentengradienten, vorzugsweise lineare Eluentengradienten, in mindestens einer oder allen Phasen verwendet werden, wobei vorzugsweise eine höhere Gradientensteigung in den Recyclingphasenschritten (IC-R und/oder B-R) als für die Reinigungsphasenschritte (IC1, B1, IC2 und/oder B2) gewählt wird.

3. Verfahren nach einem der vorhergehenden Ansprüche, wobei in miteinander verbundenen Verfahrensschritten der stromaufwärtige Abschnittseinlass über den stromaufwärtigen Adsorbereinlass mit Eluent mit einem Gradienten in Form einer sich zeitlich ändernden Modifikatorkonzentration beladen wird und wobei der aus dem stromaufwärtigen Adsorberabschnittauslass austretende Strom vor dem Eintritt in den stromabwärtigen Adsorberabschnitteinlass inline mit Eluent ohne Modifikator oder mit einer anderen Modifikatorkonzentration als am Einlass des stromaufwärtigen Adsorberabschnitts verdünnt wird,

4. Verfahren nach einem der vorhergehenden Ansprüche, wobei in Batch-Schritten des Verfahrens ohne gereinigte Produktelution der vorhergehende stromaufwärts gelegene Adsorber gereinigt wird, vorzugsweise mit Eluent mit einer höheren Modifikatorkonzentration als am Ende des vorhergehenden zusammenhängenden Recycling-Schrittes oder mit einem anderen Modifikator oder mit einer Reinigungslösung, und regeneriert wird und wobei Eluent mit einem Gradienten in Form einer sich zeitlich ändernden Modifikatorkonzentration in den vorhergehenden stromabwärts gelegenen Adsorbereinlass geladen wird
und/oder wobei in Batch-Schritten des Verfahrens mit gereinigter Produktelution der vorhergehende stromaufwärts gelegene Adsorber über den stromaufwärts gelegenen Adsorbereinlass mit Elutionsmittel mit einem Gradienten in Form einer zeitlich veränderlichen Modifikatorkonzentration beladen wird.

5. Verfahren nach einem der vorhergehenden Ansprüche, wobei in der Recycling-Phase in dem Interconnected-Recycling-Schritt (IC-R) der stromaufwärts gelegene Adsorberabschnitt über den Einlass des stromaufwärts gelegenen Adsorberabschnitts mit Eluent mit einem Gradienten in Form einer sich zeitlich ändernden Modifikatorkonzentration beladen wird, und wobei der aus dem Auslass des stromaufwärts gelegenen Adsorbers austretende Strom inline verdünnt wird, bevor er in den Einlass des stromabwärts gelegenen Adsorbers mit Eluent ohne Modifikator oder mit einer anderen Modifikatorkonzentration als am Einlass des stromaufwärts gelegenen Adsorberabschnitts gelangt;
und/oder wobei in dem optionalen Batch-Recycling-Schritt (B-R) der zuvor stromaufwärts gelegene Adsorberabschnitt des vorangegangenen Schritts a. gereinigt wird, vorzugsweise mit Eluent mit einer höheren Modifikatorkonzentration als am Ende des vorangegangenen zusammenhängenden Recyclingschritts oder mit einem anderen Modifikator oder mit einer Reinigungslösung, und regeneriert wird und wobei Eluent mit einem Gradienten in Form einer sich zeitlich ändernden Modifikatorkonzentration in den Einlass des zuvor stromabwärts gelegenen Adsorberabschnitts von Schritt a. geladen wird, um schwach adsorbierende Verunreinigungen (W) zu eluieren, soweit sie sich nicht mit Produkt (P) überlappen.

6. Verfahren nach einem der vorhergehenden Ansprüche, wobei in der Reinigungsphase im ersten zusammenhängenden Reinigungsschritt (IC1) der stromaufwärts gelegene Adsorberabschnitt über den stromaufwärts gelegenen Adsorbereinlass mit Eluent mit einem Gradienten in Form einer sich zeitlich ändernden Modifikatorkonzentration beladen wird und wobei der aus dem Auslass des stromaufwärts gelegenen Adsorberabschnitts austretende Strom vor dem Eintritt in den stromabwärts gelegenen Adsorberabschnittseinlass mit Eluent ohne Modifikator oder mit einer anderen Modifikatorkonzentration als am Einlass des stromaufwärts gelegenen Adsorberabschnitts inline verdünnt wird;
und/oder wobei in dem ersten Batch-Reinigungsschritt (B1) das Produkt (P) aus dem vorhergehenden stromaufwärts gelegenen Adsorberabschnitt eluiert wird, indem er über seinen Einlass mit Eluent mit einem Gradienten in Form einer sich zeitlich ändernden Modifikatorkonzentration beladen wird;
und/oder wobei in dem zweiten verbundenen Reinigungsschritt (IC2) der stromaufwärts gelegene Adsorberabschnittseinlass über den stromaufwärts gelegenen Adsorbereinlass mit Eluent mit einem Gradienten in Form einer sich zeitlich ändernden Modifikatorkonzentration beladen wird und wobei der Strom, der den stromaufwärts gelegenen Adsorberabschnittsauslass verlässt, inline verdünnt wird, bevor er in den stromabwärts gelegenen Adsorberabschnittseinlass mit Eluent ohne Modifikator oder mit einer anderen Modifikatorkonzentration als am Einlass des stromaufwärts gelegenen Adsorberabschnitts eintritt;
und/oder wobei in dem zweiten Batch-Reinigungsschritt (B2) der vorhergehende stromaufwärts gelegene Adsorber gereinigt wird, vorzugsweise mit Eluent mit einer höheren Modifikator-Konzentration als am Ende des vorhergehenden zusammenhängenden Recycling-Schrittes oder mit einem anderen Modifikator oder mit einer Reinigungslösung, und regeneriert wird und wobei Eluent mit einem Gradienten in Form einer sich zeitlich ändernden Modifikator-Konzentration in den vorhergehenden stromabwärts gelegenen Adsorber-Einlass geladen wird.

7. Verfahren nach einem der vorhergehenden Ansprüche 2 bis 6, wobei der Modifikator ausgewählt ist aus der Gruppe bestehend aus einem organischen oder anorganischen Lösungsmittel oder einer Mischung davon, das bzw. die sich von einem Basislösungsmittel oder einer Mischung davon des Elutionsmittels unterscheidet, einem Elektrolyten in einem solchen organischen oder anorganischen Lösungsmittel (oder einer Mischung davon), vorzugsweise ausgewählt aus einem gelösten Salz oder einem pH-Wert, oder einer Kombination davon, wobei
vorzugsweise handelt es sich bei dem Basislösungsmittel um Wasser oder ein Gemisch aus Wasser und mindestens einem organischen Lösungsmittel oder um Wasser in einem Gemisch mit einem oder mehreren Salzen und/oder organischen Lösungsmitteln, wobei eines oder beide in einem im Vergleich zu Wasser geringeren Anteil vorliegen, und wobei
weiter bevorzugt handelt es sich bei dem Modifizierungsmittel um ein organisches Lösungsmittel oder ein Gemisch aus Wasser und mindestens einem organischen Lösungsmittel mit einer höheren Konzentration des mindestens einen organischen Lösungsmittels als im Basislösungsmittel, um Wasser oder ein Gemisch aus Wasser und mindestens einem organischen Lösungsmittel mit einer anderen Salz- oder H⁺ Konzentration als im Basislösungsmittel.

8. Verfahren nach einem der vorhergehenden Ansprüche, **dadurch gekennzeichnet, dass** in mindestens einer oder allen Phasen vorzugsweise lineare Eluentengradienten mit einem Gradienten in Form einer zeitlich veränderlichen Modifikatorkonzentrationserhöhung verwendet werden.

9. Verfahren nach einem der vorhergehenden Ansprüche, wobei vor der Durchführung des ersten zusammenhängenden Recyclingschritts der ersten Recyclingphase eine Startphase durchgeführt wird, in der
während einer ersten Zeitspanne für das Anfahren der Charge (t_{B-SU-F}) werden die Adsorber (1, 2) abgeschaltet
und die Einspeisemischung (F) wird dem Einlass des Adsorberabschnitts (1) zugeführt, um der stromaufwärts gelegene Adsorberabschnitt des ersten zusammenhängenden Recyclingschritts der ersten Recyclingphase zu werden,
während der Adsorberabschnitt (2), der der nachgeschaltete Adsorberabschnitt des ersten zusammenhängenden Recyclingschritts der ersten Recyclingphase werden soll, entweder im Gleichgewicht ist oder bereits im Gleichgewicht und inaktiv ist,
und wobei vorzugsweise anschließend während einer zweiten Batch-Anfahrzeitspanne (t_{B-SU-W}) die Adsorber (1, 2) getrennt werden und eine schwach adsorbierende Verunreinigung (W) aus dem Adsorberabschnitt (1) eluiert wird, der in dem vorhergehenden ersten Batch-Anfahrschritt mit einer Beschickungsmischung (F) versorgt wurde, während der andere Adsorber (2) entweder im Gleichgewicht ist oder bereits im Gleichgewicht und inaktiv ist.

10. Verfahren nach Anspruch 9, wobei nach der ersten Chargenanlaufzeitspanne (t_{B-SU-F}) und vor der zweiten Chargenanlaufzeitspanne (t_{B-SU-W}) Elutionsmittel dem Einlass des Adsorberabschnitts zugeführt wird, um der stromaufwärts gelegene Adsorberabschnitt des ersten zusammenhängenden Recyclingschritts der ersten Recyclingphase zu werden, und wobei das Elutionsmittel ohne Modifizierungsmittel oder mit einer Modifizierungsmittelkonzentration vorliegt, die im Wesentlichen der anfänglichen Modifizierungsmittelkonzentration entspricht, die während der ersten Chargenanlaufzeitspanne oder in Abwesenheit einer zweiten Chargenanlaufzeitspanne des ersten zusammenhängenden Recyclingschritts angewendet wird;
und/oder wobei während der zweiten Batch-Anlaufzeitspanne der Einlass des Adsorberabschnitts (1), der mit dem Beschickungsgemisch (F) versorgt wurde, mit Eluent mit einem Gradienten in Form einer sich zeitlich ändernden Modifikatorkonzentration beladen wird.

11. Verfahren nach einem der vorhergehenden Ansprüche, **dadurch gekennzeichnet, dass** nach Beendigung mindestens einer Basissequenz, vorzugsweise mehr als einer Basissequenz, eine Shut-down-Phase durchgeführt wird,
wobei die Abschaltphase die folgenden Schritte in dieser Reihenfolge umfasst:
a'. einen verbundene Abschalt-Recyclingschritt (IC'-R), bei dem der stromaufwärts gelegene Adsorberabschnitt des vorhergehenden und letzten zweiten verbundenen Reinigungsschritts (IC2) die stromabwärts gelegene Position und der andere Adsorberabschnitt die stromaufwärts gelegene Position einnimmt, bei dem die Adsorberabschnitte miteinander verbunden sind und bei dem der Auslass des stromaufwärts gelegenen Adsorberabschnitts mit dem Einlass des stromabwärts gelegenen Adsorberabschnitts während einer Verbundabschalt-Recyclingzeitspanne (t_{IC'-R(-SD})) verbunden ist, wobei der stromaufwärts gelegene Adsorberabschnitt über den Einlass des stromaufwärts gelegenen Adsorberabschnitts mit Eluent beladen wird und wobei eine Fraktion, die die schwach adsorbierenden Verunreinigungen (W), zumindest soweit sie sich mit dem Produkt (P) überschneiden, das Produkt (P) und die stark adsorbierenden Verunreinigungen (S), zumindest soweit sie sich mit dem Produkt (P) überschneiden, enthält, aus dem stromaufwärts gelegenen Adsorberabschnitt in den stromabwärts gelegenen Adsorberabschnitt eluiert wird und
wobei der Strom, der den stromaufwärts gelegenen Adsorberauslass verlässt, in der Leitung verdünnt wird, bevor er in den stromabwärts gelegenen Adsorbereinlass eintritt;
b'. einen optionalen Chargenabschalt-Recyclingschritt (B'-R), in dem die Adsorberabschnitte getrennt werden, wobei die Adsorberabschnitte während einer Zeitspanne des Chargenabschalt-Recyclings (t_{B'(-R})) getrennt werden und
wobei der zuvor stromaufwärts gelegene Adsorberabschnitt des vorhergehenden Schritts a' gereinigt und regeneriert wird und wobei Elutionsmittel in den Einlass des zuvor stromabwärts gelegenen Adsorberabschnitts des Schritts a geladen wird, um schwach adsorbierende Verunreinigungen (W) zu eluieren, soweit sie sich nicht mit dem Produkt (P) überlappen;
gefolgt von den folgenden Schritten in dieser Reihenfolge, wobei der stromaufwärts gelegene Adsorberabschnitt des verbundenen Abschalt-Recyclingschritts die Funktion des stromabwärts gelegenen Adsorberabschnitts übernimmt und der stromabwärts gelegene Adsorberabschnitt des verbundenen Abschalt-Recyclingschritts die Funktion des stromaufwärts gelegenen Adsorberabschnitts übernimmt:
c'. einen ersten verbundenen Abschalt-Reinigungsschritt (IC1-SD), in dem die Adsorberabschnitte miteinander verbunden sind, wobei der stromaufwärts gelegene Auslass des Adsorberabschnitts mit dem stromabwärts gelegenen Einlass des Adsorberabschnitts während einer ersten verbundenen Abschalt-Reinigungszeitspanne (t_{IC1'}) verbunden ist,
wobei der stromaufwärts gelegene Adsorber über den stromaufwärts gelegenen Adsorbereinlass mit Eluent beladen wird und wobei eine Produktfraktion, die schwach adsorbierende Verunreinigungen (W) und Produkt (P) in Überlappung enthält, aus dem stromaufwärts gelegenen Adsorber in den stromabwärts gelegenen Adsorberabschnitt eluiert wird und
wobei der aus dem Auslass des stromaufwärts gelegenen Adsorberabschnitts austretende Strom in der Leitung verdünnt wird, bevor er in den Einlass des stromabwärts gelegenen Adsorberabschnitts eintritt
d'. einen ersten Batch-Abschalt-Reinigungsschritt (B1-SD), bei dem während einer ersten Batch-Reinigungszeitspanne (t_{B1'}) die Adsorberabschnitte getrennt werden und bei dem das Produkt (P) aus dem vorangehenden stromaufwärts gelegenen Adsorberabschnitt eluiert wird und bei dem der vorangehende stromabwärts gelegene Adsorberabschnitt entweder im Leerlauf ist oder dem Einlass des vorangehenden stromabwärts gelegenen Adsorberabschnitts Eluent zugeführt wird
e'. einen zweiten zusammenhängenden Abschalt-Reinigungsschritt (IC2-SD), bei dem der Auslass des stromaufwärts gelegenen Adsorberabschnitts mit dem Einlass des stromabwärts gelegenen Adsorberabschnitts während einer zweiten zusammenhängenden Abschalt-Reinigungszeitspanne (t_{IC2'}) verbunden ist,
wobei der stromaufwärts gelegene Adsorberabschnitt über den Einlass des stromaufwärts gelegenen Adsorberabschnitts mit Eluent beladen wird und wobei eine Produktfraktion, die Produkt (P) und stark adsorbierende Verunreinigungen (S) in Überlappung enthält, aus dem stromaufwärts gelegenen Adsorber in den stromabwärts gelegenen Adsorber eluiert wird und
wobei der aus dem Auslass des stromaufwärts gelegenen Adsorberabschnitts austretende Strom in der Leitung verdünnt wird, bevor er in den Einlass des stromabwärts gelegenen Adsorberabschnitts eintritt
f'. einen zweiten Batch-Abschalt-Reinigungsschritt (B2-SD), bei dem während einer zweiten Reinigungs-Batch-Zeitspanne (t_{B2'}) die Adsorber abgekoppelt werden und bei dem der vorherige stromaufwärts gelegene Adsorber gereinigt und regeneriert wird und bei dem Eluent in den vorherigen stromabwärts gelegenen Adsorbereinlass geladen wird
gefolgt von
g'. einen ersten endgültigen Abschaltchargenschritt (B-SD-P), bei dem während einer ersten endgültigen Abschaltchargenzeitspanne (t_{B-SD-}p) der erste und der zweite Adsorber getrennt werden und bei dem das Produkt (P) aus dem zuvor stromabwärts gelegenen Adsorber eluiert wird und bei dem der andere Adsorber im Leerlauf ist oder regeneriert wird
h'. gegebenenfalls, aber vorzugsweise einen zweiten abschließenden Abschaltchargenschritt (B-SD-S), wobei während einer zweiten abschließenden Abschaltchargenzeitspanne (t_{B-SD-S}) der erste und der zweite Adsorber getrennt werden und die stark adsorbierende Verunreinigung S aus dem zuvor stromabwärts gelegenen Adsorber eluiert wird und wobei der andere Adsorber im Leerlauf ist oder regeneriert wird.

12. Verfahren nach Anspruch 11, **dadurch gekennzeichnet, dass** in dem verbundenen Abschalt-Recyclingschritt (IC'-R), der stromaufwärts gelegene Adsorberabschnitt des vorangegangenen und letzten zweiten verbundenen Reinigungsschritts (IC2) über den Einlass des stromaufwärts gelegenen Adsorberabschnitts mit Eluent konstanter Zusammensetzung oder mit einem Gradienten in Form einer sich zeitlich ändernden Modifikatorkonzentration beladen wird, wobei der aus dem Auslass des stromaufwärts gelegenen Adsorbers austretende Strom inline verdünnt wird, bevor er in den Einlass des stromabwärts gelegenen Adsorbers mit Eluent ohne Modifikator oder mit einer anderen Modifikatorkonzentration als am Einlass des stromaufwärts gelegenen Adsorberabschnitts eintritt;
und/oder wobei in dem Batch-Abschalt-Recyclingschritt (B'-R) der zuvor stromaufwärts gelegene Adsorberabschnitt des vorhergehenden Schritts a. gereinigt und regeneriert wird und wobei Elutionsmittel mit einem Gradienten in Form einer sich zeitlich ändernden Modifikatorkonzentration in den Einlass des zuvor stromabwärts gelegenen Adsorberabschnitts des Schritts a'. geladen wird, um schwach adsorbierende Verunreinigungen (W) zu eluieren, soweit sie sich nicht mit dem Produkt (P) überlappen;
und/oder wobei in dem ersten verbundenen Shutdown-Reinigungsschritt (IC1-SD) der stromaufwärts gelegene Adsorber über den stromaufwärts gelegenen Adsorbereinlass mit einem Elutionsmittel mit einem Gradienten in Form einer sich zeitlich ändernden Modifikatorkonzentration beladen wird und wobei der Strom, der den Auslass des stromaufwärts gelegenen Adsorberabschnitts verlässt, inline verdünnt wird, bevor er in den Einlass des stromabwärts gelegenen Adsorberabschnitts mit Elutionsmittel ohne Modifikationsmittel oder mit einer anderen Modifikationsmittelkonzentration als am Einlass des stromaufwärts gelegenen Adsorberabschnitts eintritt:
und/oder wobei in dem ersten Batch-Shutdown-Reinigungsschritt (B1-SD) Produkt (P) aus dem vorhergehenden stromaufwärts gelegenen Adsorberabschnitt eluiert wird, indem über den stromaufwärts gelegenen Adsorbereinlass Elutionsmittel mit einem Gradienten in Form einer sich zeitlich ändernden Modifikatorkonzentration zugeführt wird, und wobei der vorhergehende stromabwärts gelegene Adsorberabschnitt entweder inaktiv ist oder dem vorhergehenden stromabwärts gelegenen Adsorberabschnittseinlass Elutionsmittel zugeführt wird, vorzugsweise Elutionsmittel in Form des Basislösungsmittels;
und/oder wobei in dem zweiten verbundenen Shutdown-Reinigungsschritt (IC2-SD) der stromaufwärts gelegene Adsorberabschnitt über den Einlass des stromaufwärts gelegenen Adsorberabschnitts mit Eluent konstanter Zusammensetzung oder mit einem Gradienten in Form einer sich zeitlich ändernden Modifikatorkonzentration beladen wird und wobei der den Auslass des stromaufwärts gelegenen Adsorberabschnitts verlassende Strom inline verdünnt wird, bevor er in den Einlass des stromabwärts gelegenen Adsorberabschnitts mit Eluent ohne Modifikator oder mit einer anderen Modifikatorkonzentration als am Einlass des stromaufwärts gelegenen Adsorberabschnitts eintritt;
und/oder wobei in dem zweiten Batch-Shutdown-Reinigungsschritt (B2-SD) der vorherige stromaufwärts gelegene Adsorber gereinigt und regeneriert wird und wobei Eluent in den vorherigen stromabwärts gelegenen Adsorbereinlass geladen wird;
und/oder wobei in dem ersten abschließenden Abschalt-Batch-Schritt (B-SD-P) das Produkt (P) aus dem zuvor stromabwärts gelegenen Adsorber mit einem Elutionsmittel mit einem Gradienten in Form einer sich zeitlich ändernden Modifikatorkonzentration eluiert wird und wobei der andere Adsorber inaktiv ist oder regeneriert wird;
und/oder wobei in dem ersten abschließenden zweiten abschließenden Abschalt-Batch-Schritt (B-SD-S) die stark adsorbierende Verunreinigung S aus dem zuvor stromabwärts gelegenen Adsorber eluiert wird und wobei der andere Adsorber inaktiv oder regeneriert ist.

13. Verfahren nach einem der vorhergehenden Ansprüche, wobei in der Recyclingphase und/oder in der Reinigungsphase lineare Gradienten mit unterschiedlichen Steigungen und/oder Flussraten verwendet werden.

14. Verfahren nach einem der vorhergehenden Ansprüche 2-13, wobei in den meisten oder in allen miteinander verbundenen Schritten des Verfahrens der stromaufwärts gelegene Abschnittseinlass über den stromaufwärts gelegenen Adsorbereinlass mit Elutionsmittel mit einem Gradienten in Form einer sich zeitlich ändernden Modifikatorkonzentration beladen wird und wobei der aus dem stromaufwärts gelegenen Adsorberabschnittsauslass austretende Strom inline verdünnt wird, bevor er in den stromabwärts gelegenen Adsorberabschnittseinlass mit Elutionsmittel ohne Modifikationsmittel oder mit einer anderen Modifikationsmittelkonzentration als am Einlass des stromaufwärts gelegenen Adsorberabschnitts eintritt,
und wobei
in Batch-Schritten des Verfahrens ohne gereinigte Produktelution der vorhergehende stromaufwärts gelegene Adsorber mit Eluent gereinigt und regeneriert wird und gegebenenfalls Eluent mit einem Gradienten in Form einer sich zeitlich ändernden Modifikatorkonzentration in den vorhergehenden stromabwärts gelegenen Adsorbereinlass geladen wird oder bei Batch-Schritten des Verfahrens mit gereinigter Produktelution der vorgeschaltete Adsorber über den vorgeschalteten Adsorbereinlass mit Elutionsmittel mit einem Gradienten in Form einer zeitlich veränderlichen Modifikatorkonzentration beladen wird
und wobei der Modifikator aus der Gruppe ausgewählt ist, die aus einem Lösungsmittel oder einem Gemisch davon, das sich von einem Basislösungsmittel oder einem Gemisch davon des Elutionsmittels unterscheidet, einem Elektrolyten in einem solchen Lösungsmittel oder einem Gemisch davon, vorzugsweise ausgewählt aus einem gelösten Salz oder einem pH-Wert, oder einer Kombination davon besteht, wobei das Basislösungsmittel vorzugsweise Wasser oder ein Gemisch von Wasser mit mindestens einem organischen Lösungsmittel oder Wasser in einem Gemisch mit einem oder mehreren Salzen und/oder organischen Lösungsmitteln in einem geringeren Anteil im Vergleich zu Wasser ist, und wobei ferner vorzugsweise der Modifikator ein organisches Lösungsmittel oder ein Gemisch von Wasser mit mindestens einem organischen Lösungsmittel mit einer höheren Konzentration des mindestens einen organischen Lösungsmittels als das Basislösungsmittel, Wasser oder ein Gemisch von Wasser mit einer anderen Salz- oder H⁺ Konzentration als das Basislösungsmittel ist,
und wobei die Modifikatorkonzentration im Verfahren von einer niedrigsten Konzentration zu Beginn der Elution (B-SU-W, B-R, B'-R, B2, B2-SD) der schwach adsorbierenden Verunreinigungen (W), soweit sie sich nicht mit dem Produkt (P) überlappt, über die folgenden Schritte erhöht, vorzugsweise linear erhöht wird
Recycling (IC-R, IC'-R, IC1, IC1-SD), außer im Falle eines nachfolgenden abschließenden Chargenschritts (B-SD-P) und
gegebenenfalls einer Produktelution (B1, B1-SD, B-SD-P) und gegebenenfalls einer weiteren Rückführung (IC2, IC2-SD), wobei während der Rückführung (IC2, IC2-SD) die Modifikatorkonzentration variiert oder nur für die Rückführung (IC2, IC2-SD) konstant gehalten werden kann
bis zu einer höchsten Konzentration vor Beginn der Elution (B-R, B'-R, B2, B2-SD, B-SD-S) der stark adsorbierenden Verunreinigungen (S).

15. Verwendung eines Verfahrens nach einem der vorhergehenden Ansprüche zur Reinigung von Biomolekülen natürlichen oder synthetischen Ursprungs, vorzugsweise ausgewählt aus der Gruppe bestehend aus Nukleinsäuremolekülen, einschließlich DNA-und RNA-Molekülen, Proteinen, einschließlich Antikörpern, Peptiden, Kohlenhydraten, Lipiden sowie Kombinationen und Modifikationen sowie Fragmenten davon.

## Revendications

1. Méthode de purification par chromatographie cyclique pour l'isolement d'un produit (P) à partir d'un mélange d'alimentation (F) constitué du produit (P) et d'au moins deux autres composants représentant des impuretés faiblement adsorbantes (W) et des impuretés fortement adsorbantes (S),
la méthode utilisant seulement deux sections d'adsorption chromatographique comme phase stationnaire chromatographique, dans laquelle une première section d'adsorption (1) a une première entrée de section d'adsorption et une première sortie de section d'adsorption, et une deuxième section d'adsorption (2) a une deuxième entrée de section d'adsorption et une deuxième sortie de section d'adsorption,
dans laquelle la méthode comprend au moins une séquence de base ayant au moins une phase de recyclage suivie d'une seule phase de purification, les séquences de base se répétant de préférence au moins deux fois de manière cyclique,
et dans laquelle ladite phase de recyclage consiste en au moins une séquence de recyclage comprenant les étapes suivantes :
a. une étape de recyclage interconnecté (IC-R), au cours de laquelle les sections d'adsorption sont interconnectées et où une sortie de section d'adsorption en amont est connectée à une entrée de section d'adsorption en aval pendant une période de recyclage interconnecté [t_{IC-R}],
la section d'adsorption en amont est chargée d'éluant via l'entrée de la section d'adsorption en amont et une fraction contenant les impuretés faiblement adsorbantes (W) au moins dans la mesure où elles se chevauchent avec le produit (P), le produit (P) et les impuretés fortement adsorbantes (S) au moins dans la mesure où elles se chevauchent avec le produit (P) est éluée de la section d'adsorption en amont dans la section d'adsorption en aval, et
dans lequel le flux sortant de la sortie de l'adsorbeur en amont est dilué en ligne avant d'entrer dans l'entrée de l'adsorbeur en aval ;
b. une étape facultative de recyclage par lots (B-R), dans laquelle, au cours d'une période de recyclage par lots (t_{B(-R})), lesdites sections de l'adsorbeur sont déconnectées et
dans lequel la section adsorbante en amont de l'étape a. précédente est nettoyée pour éliminer les impuretés fortement adsorbantes (S) et régénérée et dans lequel l'éluant est chargé à l'entrée de la section adsorbante en aval de l'étape a. pour éluer les impuretés faiblement adsorbantes (W) jusqu'à ce qu'elles ne se superposent pas au produit (P) ;
après chaque séquence de recyclage des étapes a. et b., les sections de l'adsorbeur sont commutées dans l'ordre, le nombre de séquences de recyclage étant ≥ 1,
et dans lequel chaque phase de recyclage est suivie d'une seule phase de purification,
phase de purification comprend les étapes suivantes dans l'ordre, la section d'adsorption en amont de la dernière étape de recyclage interconnectée de la phase de recyclage précédente jouant le rôle de la section d'adsorption en aval et la section d'adsorption en aval de la dernière étape de recyclage interconnectée de la phase de recyclage précédente jouant le rôle de la section d'adsorption en amont :
c. une première étape de purification interconnectée (IC1), au cours de laquelle les sections d'adsorption sont interconnectées, la sortie de la section d'adsorption en amont étant connectée à l'entrée de la section d'adsorption en aval pendant une première période de purification interconnectée (t_{IC1}),
dans lequel l'adsorbeur en amont est chargé d'éluant via l'entrée de l'adsorbeur en amont et dans lequel une fraction de produit contenant des impuretés faiblement adsorbantes (W) et du produit (P) en chevauchement est éluée de l'adsorbeur en amont dans la section de l'adsorbeur en aval, et
dans lequel le flux sortant de la sortie de la section d'adsorption en amont est dilué en ligne avant d'entrer dans l'entrée de la section d'adsorption en aval
d. une première étape de purification par lots (B1), dans laquelle, pendant une première période de purification par lots (t_{B1}), lesdites sections d'adsorption sont déconnectées et dans laquelle le produit (P) est élué de la section d'adsorption précédente en amont et dans laquelle le mélange d'alimentation (F) est fourni à l'entrée de la section d'adsorption précédente en aval
e. une deuxième étape de purification interconnectée (IC2), dans laquelle la sortie de la section d'adsorption en amont est connectée à l'entrée de la section d'adsorption en aval pendant une deuxième période de purification interconnectée (t_{IC2}),
la section d'adsorption en amont est chargée d'éluant par l'entrée de la section d'adsorption en amont et une fraction de produit contenant du produit (P) et des impuretés fortement adsorbantes (S) en chevauchement est éluée de l'adsorbeur en amont dans l'adsorbeur en aval et
dans lequel le flux sortant de la sortie de la section d'adsorption en amont est dilué en ligne avant d'entrer dans l'entrée de la section d'adsorption en aval
f. une deuxième étape de purification par lots (B2), dans laquelle, au cours d'une deuxième période de purification par lots (t_{B2}), lesdits adsorbeurs sont déconnectés, l'adsorbeur amont précédent est nettoyé et régénéré et l'éluant est chargé à l'entrée de l'adsorbeur aval précédent.

2. Méthode selon la revendication 1, dans laquelle des gradients d'éluant, de préférence des gradients d'éluant linéaires, sont utilisés dans au moins une ou toutes les phases, une pente de gradient plus élevée étant sélectionnée dans les étapes de la phase de recyclage (IC-R et/ou B-R) que pour les étapes de la phase de purification (IC1, B1, IC2, et/ou B2).

3. Méthode selon l'une quelconque des revendications précédentes, dans laquelle, au cours d'étapes interconnectées de la méthode, l'entrée de la section amont est chargée, via l'entrée de l'adsorbeur amont, d'un éluant avec un gradient sous la forme d'une concentration de modificateur variant dans le temps et dans laquelle le flux sortant de la sortie de la section adsorbante amont est dilué en ligne avant d'entrer dans l'entrée de la section adsorbante aval avec un éluant sans modificateur ou avec une concentration de modificateur différente de celle de l'entrée de la section adsorbante amont,

4. Procédé selon l'une quelconque des revendications précédentes, dans lequel, dans les étapes discontinues du procédé sans élution de produit purifié, l'adsorbeur amont précédent est nettoyé, de préférence avec un éluant dont la concentration en modificateur est plus élevée qu'à la fin de l'étape de recyclage interconnectée précédente ou avec un modificateur différent ou avec une solution de nettoyage, et régénéré, et dans lequel l'éluant avec un gradient sous la forme d'une concentration en modificateur variant dans le temps est chargé à l'entrée de l'adsorbeur aval précédent.
et/ou dans lequel, dans les étapes discontinues de la méthode avec élution du produit purifié, l'adsorbeur amont précédent est chargé, via l'entrée de l'adsorbeur amont, d'un éluant avec un gradient sous la forme d'une concentration de modificateur variant dans le temps.

5. Procédé selon l'une quelconque des revendications précédentes, dans lequel, dans ladite phase de recyclage, dans ladite étape de recyclage interconnectée (IC-R), la section adsorbante en amont est chargée, via l'entrée de la section adsorbante en amont, d'un éluant avec un gradient sous la forme d'une concentration de modificateur changeant dans le temps, et dans lequel le flux sortant de la sortie de l'adsorbeur en amont est dilué en ligne avant d'entrer dans l'entrée de l'adsorbeur en aval avec un éluant sans modificateur ou avec une concentration de modificateur différente de celle de l'entrée de la section adsorbante en amont ;
et/ou dans lequel, dans l'étape facultative de recyclage par lots (B-R), la section adsorbante précédemment en amont de l'étape précédente a. est nettoyée, de préférence avec un éluant ayant une concentration de modificateur plus élevée qu'à la fin de l'étape de recyclage interconnectée précédente ou avec un modificateur différent ou avec une solution de nettoyage, et régénérée, et dans laquelle un éluant avec un gradient sous la forme d'une concentration de modificateur changeant dans le temps est chargé à l'entrée de la section adsorbante précédemment en aval de l'étape a. pour éluer les impuretés faiblement adsorbantes (W) dans la mesure où elles ne se chevauchent pas avec le produit (P).

6. Procédé selon l'une quelconque des revendications précédentes, dans lequel, dans ladite phase de purification, lors de la première étape de purification interconnectée (IC1), la section adsorbante en amont est chargée, via l'entrée de l'adsorbeur en amont, d'un éluant présentant un gradient sous la forme d'une concentration de modificateur variant dans le temps, et dans lequel le flux sortant de la sortie de la section adsorbante en amont est dilué en ligne avant d'entrer dans l'entrée de la section adsorbante en aval avec un éluant sans modificateur ou avec une concentration de modificateur différente de celle de l'entrée de la section adsorbante en amont ;
et/ou dans lequel, dans ladite première étape de purification par lots (B1), le produit (P) est élué de la section adsorbante précédente en amont en la chargeant, via son entrée, d'un éluant avec un gradient sous la forme d'une concentration de modificateur changeant dans le temps ;
et/ou dans laquelle, lors de la deuxième étape de purification interconnectée (IC2), l'entrée de la section adsorbante en amont est chargée, via l'entrée de l'adsorbeur en amont, d'un éluant avec un gradient sous la forme d'une concentration de modificateur changeant dans le temps et dans laquelle le flux sortant de la sortie de la section adsorbante en amont est dilué en ligne avant d'entrer dans l'entrée de la section adsorbante en aval avec un éluant sans modificateur ou avec une concentration de modificateur différente de celle de l'entrée de la section adsorbante en amont ;
et/ou dans lequel, lors de la deuxième étape de purification par lots (B2), l'adsorbeur amont précédent est nettoyé, de préférence avec un éluant dont la concentration en modificateur est plus élevée qu'à la fin de l'étape de recyclage interconnectée précédente ou avec un modificateur différent ou avec une solution de nettoyage, et régénéré, et dans lequel l'éluant avec un gradient sous la forme d'une concentration en modificateur variant dans le temps est chargé à l'entrée de l'adsorbeur aval précédent.

7. Méthode selon l'une des revendications précédentes 2-6, dans laquelle le modificateur est choisi dans le groupe constitué d'un solvant organique ou inorganique ou d'un mélange de ceux-ci différent d'un solvant de base ou d'un mélange de ceux-ci de l'éluant, d'un électrolyte dans un tel solvant organique ou inorganique (ou un mélange de ceux-ci), de préférence choisi parmi un sel dissous ou un pH, ou d'une combinaison de ceux-ci, dans laquelle
De préférence, ledit solvant de base est de l'eau ou un mélange d'eau et d'au moins un solvant organique ou de l'eau dans un mélange avec un ou plusieurs sels et/ou solvants organiques, l'un ou l'autre ou les deux dans une proportion mineure par rapport à l'eau, et dans lequel
De préférence, ce modificateur est un solvant organique ou un mélange d'eau et d'au moins un solvant organique dont la concentration en solvant organique est supérieure à celle du solvant de base, de l'eau ou un mélange d'eau et d'au moins un solvant organique dont la concentration en sel ou en H⁺ est différente de celle du solvant de base.

8. Méthode selon l'une quelconque des revendications précédentes, dans laquelle des gradients d'éluant, de préférence linéaires, sont utilisés dans au moins une ou toutes les phases, avec un gradient sous la forme d'une augmentation de la concentration du modificateur variant dans le temps.

9. Procédé selon l'une quelconque des revendications précédentes, dans lequel, avant d'effectuer la première étape de recyclage interconnectée de la première phase de recyclage, on procède à une phase de démarrage au cours de laquelle
pendant une première période de démarrage du lot (t_{B(-SU-F})), lesdits adsorbeurs (1,2) sont déconnectés
et le mélange d'alimentation (F) est fourni à l'entrée de la section adsorbante (1) pour devenir la section adsorbante en amont de la première étape de recyclage interconnectée de la première phase de recyclage,
tandis que la section adsorbante (2) qui deviendra la section adsorbante en aval de la première étape de recyclage interconnectée de la première phase de recyclage est soit en cours d'équilibrage, soit déjà équilibrée et inactive,
et dans lequel, de préférence, par la suite, pendant une deuxième période de démarrage du lot (t_{B(-SU-W})), lesdits adsorbeurs (1,2) sont déconnectés et l'impureté faiblement adsorbante (W) est éluée de la section adsorbante (1) qui a été alimentée avec le mélange d'alimentation (F) lors de la première étape précédente de démarrage du lot, tandis que l'autre adsorbeur (2) est soit en cours d'équilibrage, soit déjà équilibré et inactif.

10. Procédé selon la revendication 9, dans lequel, après le temps de démarrage du premier lot (t_{B(-SU-F})) et avant le temps de démarrage du deuxième lot (t_{B(-SU-W})), l'éluant est fourni à l'entrée de la section adsorbante pour devenir la section adsorbante en amont de la première étape de recyclage interconnectée de la première phase de recyclage, et dans lequel ledit éluant est sans modificateur ou avec une concentration de modificateur correspondant essentiellement à la concentration de départ du modificateur appliquée pendant ladite période de démarrage du premier lot ou en l'absence d'une période de démarrage du deuxième lot de la première étape de recyclage interconnectée ;
et/ou dans lequel, pendant cette deuxième période de démarrage du lot, l'entrée de la section adsorbante (1) ayant été alimentée par le mélange d'alimentation (F) est chargée d'éluant avec un gradient sous la forme d'une concentration de modificateur changeant dans le temps.

11. Méthode selon l'une des revendications précédentes, dans laquelle une phase d'arrêt est exécutée après la fin d'au moins une séquence de bases, de préférence plus d'une séquence de bases,
dans laquelle la phase d'arrêt comprend les étapes suivantes dans l'ordre :
a'. une étape interconnectée de recyclage à l'arrêt (IC'-R), dans laquelle la section d'adsorption en amont de la précédente et dernière deuxième étape interconnectée de purification (IC2) prend la position aval et l'autre section d'adsorption prend la position amont, dans laquelle les sections d'adsorption sont interconnectées et dans laquelle la sortie de la section d'adsorption en amont est connectée à l'entrée de la section d'adsorption en aval pendant une période interconnectée de recyclage à l'arrêt [t_{IC'(-R)(-SD)}],
la section d'adsorption en amont est chargée d'éluant via l'entrée de la section d'adsorption en amont et une fraction contenant les impuretés faiblement adsorbantes (W) au moins dans la mesure où elles se chevauchent avec le produit (P), le produit (P) et les impuretés fortement adsorbantes (S) au moins dans la mesure où elles se chevauchent avec le produit (P) est éluée de la section d'adsorption en amont dans la section d'adsorption en aval, et
dans lequel le flux sortant de la sortie de l'adsorbeur en amont est dilué en ligne avant d'entrer dans l'entrée de l'adsorbeur en aval ;
b'. une étape facultative de recyclage par arrêt du lot (B'-R), au cours de laquelle les sections d'adsorption sont déconnectées, dans laquelle, pendant une période de temps du lot de recyclage par arrêt (t_{B'(-R})), lesdites sections d'adsorption sont déconnectées et
dans lequel la section adsorbante en amont de l'étape a' précédente est nettoyée et régénérée et dans lequel l'éluant est chargé à l'entrée de la section adsorbante en aval de l'étape a pour éluer les impuretés faiblement adsorbantes (W) dans la mesure où elles ne se superposent pas au produit (P) ;
suivi des étapes suivantes dans l'ordre, la section d'adsorption en amont de l'étape de recyclage avec arrêt interconnecté jouant le rôle de la section d'adsorption en aval et la section d'adsorption en aval de l'étape de recyclage avec arrêt interconnecté jouant le rôle de la section d'adsorption en amont :
c'. une première étape de purification par arrêt interconnecté (IC1-SD), au cours de laquelle les sections d'adsorption sont interconnectées, la sortie de la section d'adsorption en amont étant connectée à l'entrée de la section d'adsorption en aval pendant une première période de purification par arrêt interconnecté (t_{IC1'}),
dans lequel l'adsorbeur en amont est chargé d'éluant via l'entrée de l'adsorbeur en amont et dans lequel une fraction de produit contenant des impuretés faiblement adsorbantes (W) et du produit (P) en chevauchement est éluée de l'adsorbeur en amont dans la section de l'adsorbeur en aval, et
dans lequel le flux sortant de la sortie de la section d'adsorption en amont est dilué en ligne avant d'entrer dans l'entrée de la section d'adsorption en aval
d'. une première étape de purification avec arrêt du lot (B1-SD), dans laquelle, pendant une première période de purification du lot (t_{B1('})), lesdites sections d'adsorption sont déconnectées et dans laquelle le produit (P) est élué de la section d'adsorption précédente en amont et dans laquelle la section d'adsorption précédente en aval est soit inactive, soit l'éluant est fourni à l'entrée de la section d'adsorption précédente en aval.
e'. une deuxième étape de purification par arrêt interconnecté (IC2-SD), dans laquelle la sortie de la section d'adsorption en amont est connectée à l'entrée de la section d'adsorption en aval pendant une deuxième période de purification par arrêt interconnecté (t_{IC2'}),
la section d'adsorption en amont est chargée d'éluant par l'entrée de la section d'adsorption en amont et une fraction de produit contenant du produit (P) et des impuretés fortement adsorbantes (S) en chevauchement est éluée de l'adsorbeur en amont dans l'adsorbeur en aval et
dans lequel le flux sortant de la sortie de la section d'adsorption en amont est dilué en ligne avant d'entrer dans l'entrée de la section d'adsorption en aval
f'. une deuxième étape de purification avec arrêt du lot (B2-SD), dans laquelle, au cours d'une deuxième période de purification du lot (t_{B2('})), lesdits adsorbeurs sont déconnectés, l'adsorbeur amont précédent est nettoyé et régénéré et l'éluant est chargé à l'entrée de l'adsorbeur aval précédent.
suivi de
g'. une première étape d'arrêt final du lot (B-SD-P), dans laquelle, pendant une première période d'arrêt final du lot (t_{B(-SD-P})), lesdits premier et second adsorbeurs sont déconnectés et dans laquelle le produit (P) est élué de l'adsorbeur situé précédemment en aval et dans laquelle l'autre adsorbeur est inactif ou régénéré.
h'. éventuellement, mais de préférence, une deuxième étape d'arrêt final du lot (B-SD-S), dans laquelle, au cours d'une deuxième période d'arrêt final du lot (t_{B(-SD-S})), lesdits premier et deuxième adsorbeurs sont déconnectés et l'impureté S fortement adsorbée est éluée de l'adsorbeur situé précédemment en aval, et dans laquelle l'autre adsorbeur est inutilisé ou régénéré.

12. Procédé selon la revendication 11, dans lequel, dans ladite étape interconnectée de recyclage à l'arrêt (IC'-R), la section adsorbante en amont de la précédente et dernière deuxième étape de purification interconnectée (IC2) est chargée, via l'entrée de la section adsorbante en amont, d'un éluant de composition constante ou d'un gradient sous la forme d'une concentration de modificateur changeant dans le temps, le flux sortant de la sortie de l'adsorbeur en amont étant dilué en ligne avant d'entrer dans l'entrée de l'adsorbeur en aval avec un éluant sans modificateur ou avec une concentration de modificateur différente de celle de l'entrée de la section adsorbante en amont ;
et/ou dans lequel, dans ladite étape de recyclage par arrêt discontinu (B'-R), la section adsorbante précédemment en amont de l'étape a. précédente est nettoyée et régénérée et dans lequel l'éluant avec un gradient sous la forme d'une concentration de modificateur changeant dans le temps est chargé à l'entrée de la section adsorbante précédemment en aval de l'étape a'. pour éluer les impuretés faiblement adsorbantes (W) dans la mesure où elles ne se chevauchent pas avec le produit (P) ;
et/ou dans lequel, lors de la première étape de purification à l'arrêt interconnectée (IC1-SD), l'adsorbeur en amont est chargé, via l'entrée de l'adsorbeur en amont, d'un éluant avec un gradient sous la forme d'une concentration de modificateur changeant dans le temps et dans lequel le flux sortant de la sortie de la section de l'adsorbeur en amont est dilué en ligne avant d'entrer dans l'entrée de la section de l'adsorbeur en aval avec un éluant sans modificateur ou avec une concentration de modificateur différente de celle de l'entrée de la section de l'adsorbeur en amont :
et/ou dans lequel, dans ladite première étape de purification par arrêt du lot (B1-SD), le produit (P) est élué de la section adsorbante précédente en amont par chargement, via l'entrée de l'adsorbeur en amont, d'un éluant avec un gradient sous la forme d'une concentration de modificateur changeant dans le temps et dans lequel la section adsorbante précédente en aval est soit inactive, soit l'éluant est fourni à l'entrée de la section adsorbante précédente en aval, de préférence l'éluant sous la forme d'un solvant de base ;
et/ou dans lequel, lors de la deuxième étape de purification à l'arrêt interconnectée (IC2-SD), la section adsorbante en amont est chargée, via l'entrée de la section adsorbante en amont, d'un éluant de composition constante ou d'un gradient sous la forme d'une concentration de modificateur changeant dans le temps, et dans lequel le flux sortant de la sortie de la section adsorbante en amont est dilué en ligne avant d'entrer dans l'entrée de la section adsorbante en aval avec un éluant sans modificateur ou avec une concentration de modificateur différente de celle de l'entrée de la section adsorbante en amont ;
et/ou dans lequel, au cours de la deuxième étape de purification par arrêt du lot (B2-SD), l'adsorbeur amont précédent est nettoyé et régénéré et dans lequel l'éluant est chargé à l'entrée de l'adsorbeur aval précédent ;
et/ou dans lequel, lors de la première étape d'arrêt final du lot (B-SD-P), le produit (P) est élué de l'adsorbeur situé précédemment en aval avec un éluant présentant un gradient sous la forme d'une concentration de modificateur variant dans le temps, et dans lequel l'autre adsorbeur est inutilisé ou régénéré ;
et/ou dans lequel, au cours de la première étape finale d'arrêt du lot (B-SD-S), l'impureté S fortement adsorbée est éluée de l'adsorbeur situé en aval et l'autre adsorbeur est inutilisé ou régénéré.

13. Méthode selon l'une des revendications précédentes, dans laquelle des gradients linéaires ayant des pentes et/ou des débits différents sont utilisés dans la phase de recyclage et/ou dans la phase de purification.

14. Méthode selon l'une des revendications précédentes 2-13, dans laquelle, dans la plupart ou dans toutes les étapes interconnectées de la méthode, l'entrée de la section amont est chargée, via l'entrée de l'adsorbeur amont, d'un éluant avec un gradient sous la forme d'une concentration de modificateur changeant dans le temps, et dans laquelle le flux sortant de la sortie de la section adsorbante amont est dilué en ligne avant d'entrer dans l'entrée de la section adsorbante aval avec un éluant sans modificateur ou avec une concentration de modificateur différente de celle de l'entrée de la section adsorbante amont,
et dans lequel
dans les étapes discontinues de la méthode sans élution de produit purifié, l'adsorbeur amont précédent est nettoyé avec de l'éluant et régénéré et, le cas échéant, l'éluant avec un gradient sous la forme d'une concentration de modificateur changeant dans le temps est chargé à l'entrée de l'adsorbeur aval précédent
soit, dans les étapes discontinues de la méthode avec élution du produit purifié, l'adsorbeur amont précédent est chargé, via l'entrée de l'adsorbeur amont, d'un éluant avec un gradient sous la forme d'une concentration de modificateur changeant dans le temps.
et dans lequel le modificateur est choisi dans le groupe constitué d'un solvant ou d'un mélange de solvants différent d'un solvant de base ou d'un mélange de solvants de l'éluant, d'un électrolyte dans un tel solvant ou mélange de solvants, de préférence choisi parmi un sel dissous ou un pH, ou une combinaison de ceux-ci, dans lequel de préférence ledit solvant de base est de l'eau ou un mélange d'eau avec au moins un solvant organique ou de l'eau dans un mélange avec un ou plusieurs sels et/ou solvants organiques dans une proportion mineure par rapport à l'eau, et dans lequel, de préférence, ledit modificateur est un solvant organique ou un mélange d'eau avec au moins un solvant organique ayant une concentration plus élevée dudit au moins un solvant organique que le solvant de base, de l'eau ou un mélange d'eau avec une concentration de sel ou de H⁺ différente de celle du solvant de base,
et dans lequel ladite concentration du modificateur est augmentée, de préférence de manière linéaire, au cours du processus à partir d'une concentration la plus faible au début de l'élution (B-SU-W, B-R, B'-R, B2, B2-SD) des impuretés faiblement adsorbantes (W) jusqu'à ce qu'elles ne se superposent pas au produit (P), au cours des étapes suivantes de
recyclage (IC-R, IC'-R, IC1, IC1-SD), sauf dans le cas d'une étape suivante d'arrêt final du lot (B-SD-P), et
cas échéant, élution du produit (B1, B1-SD, B-SD-P) et, le cas échéant, recyclage supplémentaire (IC2, IC2-SD), la concentration du modificateur pouvant varier au cours dudit recyclage (IC2, IC2-SD) ou être maintenue constante uniquement pour le recyclage (IC2, IC2-SD)
à une concentration maximale avant le début de l'élution (B-R, B'-R, B2, B2-SD, B-SD-S) des impuretés fortement adsorbantes (S).

15. Utilisation d'une méthode selon l'une quelconque des revendications précédentes pour la purification de biomolécules, d'origine naturelle ou synthétique, de préférence choisies dans le groupe constitué par les molécules d'acide nucléique, y compris les molécules d'ADN et d'ARN, les protéines, y compris les anticorps, les peptides, les hydrates de carbone, les lipides ainsi que les combinaisons et les modifications et les fragments de ceux-ci.
